# EUROPEAN PATENT APPLICATION

(11) **EP 1 970 078 A1**
(43) Date of publication of application: **17.09.2008**
(21) Application number: 07706535.7
(22) Date of filing: 10.01.2007
(51) Int. Cl.: A61K 48/00, A61K 9/127, A61K 31/7105, A61K 47/10, A61K 47/18, A61K 47/24, A61K 47/28, A61K 47/44, A61P 27/02

(54) **COMPOSITION INHIBITING THE EXPRESSION OF TARGET GENE IN EYEBALL AND REMEDY FOR DISEASE IN EYEBALL**

(30) Priority: 11.01.2006 JP 2006004225; 12.04.2006 JP 2006110190
(71) Applicant: KYOWA HAKKO KOGYO CO., LTD., Chiyoda-ku, Tokyo 100-8185 (JP); The University of Tokyo, Bunkyo-Ku, Tokyo 113-8654 (JP)
(72) Inventor: YAGI, Nobuhiro, c/o Kyowa Hakko Kogyo Co.,Ltd., Sunto-gun, Shizuoka 4118731 (JP); YAMAUCHI, Masahiro, West Lafayette, IN 47906 (US); AOKI, Noboru, Kyowa Hakko Kogyo Co.,Ltd., Sunto-gun, Shizuoka 411-8731 (JP); TAMAKI, Yasuhiro, c/o The University of Tokyo, Tokyo 1138654 (JP); TAKAHASHI, Hidenori, c/o The University of Tokyo, Tokyo 1138654 (JP); IRIYAMA, Aya, c/o The University of Tokyo, Tokyo 1138654 (JP); NAGAI, Ryozo, c/o The University of Tokyo, Tokyo 1138654 (JP); MANABE, Ichiro, c/o The University of Tokyo, Tokyo 1138654 (JP)
(74) Representative: Vossius & Partner
(86) International application number: PCT/JP2007/050188
(87) International publication number: WO 2007/080902

(57) **Abstract**

There is provided a composition comprising an RNA-encapsulated liposome, the RNA comprising a sequence being of 15 to 30 contiguous nucleotides of mRNA of a target gene associated with a disease in an eyeball, and a sequence complementary to the sequence, wherein the liposome is capable of reaching an eyeball, and the like. The liposome in which an RNA is encapsulated is, for example, a liposome composing a complex particle comprising a lead particle and the RNA as constituent components, the lead particle comprising a cationic lipid, and a bilayer lipid membrane for coating the complex particle. The bilayer lipid membrane is a bilayer lipid membrane comprising a neutral lipid, and a lipid derivative, a fatty acid derivative or an aliphatic hydrocarbon derivative of a water-soluble substance as constituent components.

SEQUENCE LISTING
<110> KYOWA HAKKO KOGYO CO., LTD
      THE UNIVERSITY OF TOKYO

<120> Compositions acting to suppress the target gene expression in the eyeball
and agents for the treatment of the eye disorders

<130> K04F2165
<150> JP2006-110190, JP2006-4225
<151> 2006-4-12, 2006-1-11

<160> 28

<210> 1
<211> 21
<212> RNA
<213> Artificial Sequence

<400> 1
caugaacguc uuccucccut t                                                21

<210> 2
<211> 21
<212> RNA
<213> Artificial Sequence

<400> 2
agggaggaag acguucaugt t                                                21

<210> 3
<211> 21
<212> RNA
<213> Artificial Sequence

<400> 3
auuuaccugc cacucugccu u                                                21

<210> 4
<211> 21
<212> RNA
<213> Artificial Sequence

<400> 4
ggcagagugg cagguaaauu u                                                21

<210> 5
<211> 21
<212> RNA
<213> Artificial Sequence

<400> 5
ggaguaaccc ggaucuggau u                                                21

<210> 6
<211> 21
<212> RNA
<213> Artificial Sequence

<400> 6
uccagauccg gguuacuccu u                                                21

<210> 7
<211> 21
<212> RNA
<213> Artificial Sequence

<400> 7
aagcucaccu gaggacucau u                                                21

<210> 8
<211> 21
<212> RNA
<213> Artificial Sequence

<400> 8
ugaguccuca ggugagcuuu u                                                21

<210> 9
<211> 21
<212> RNA

<213> Artificial Sequence

<400> 9
uccccagacc guccaugccu u                                                21

<210> 10
<211> 21
<212> RNA
<213> Artificial Sequence

<400> 10
ggcauggacg gucuggggu u                                                 21

<210> 11
<211> 21
<212> RNA
<213> Artificial Sequence

<400> 11
cgcugcgccc acccgccugu u                                                21

<210> 12
<211> 21
<212> RNA
<213> Artificial Sequence

<400> 12
caggcgggug ggcgcagcgu u                                                21

<210> 13
<211> 21
<212> RNA
<213> Artificial Sequence

<400> 13
auggagaagu aucugacccu u                                                21

<210> 14
<211> 21
<212> RNA
<213> Artificial Sequence

<400> 14
gggucagaua cuucuccauu u                                                21

<210> 15
<211> 21
<212> RNA
<213> Artificial Sequence

<400> 15
aguauagacg agacagugcu u                                                21

<210> 16
<211> 21
<212> RNA
<213> Artificial Sequence

<400> 16
gcacugucuc gucuauacuu u                                                21

<210> 17
<211> 21
<212> RNA
<213> Artificial Sequence

<400> 17
accagacggc aguaauggau u                                                21

<210> 18
<211> 21
<212> RNA
<213> Artificial Sequence

<400> 18
uccauuacug ccgucuggcu u                                                21

<210> 19
<211> 21
<212> RNA

<213> Artificial Sequence

<400> 19
gcucagagcc uggaaguccu u                                                21

<210> 20
<211> 21
<212> RNA
<213> Artificial Sequence

<400> 20
ggacuuccag gcucugagcu u                                                21

<210> 21
<211> 21
<212> RNA
<213> Artificial Sequence

<400> 21
gccguuccag ugcauggugu u                                                21

<210> 22
<211> 21
<212> RNA
<213> Artificial Sequence

<400> 22
caccaugcac uggaacggcu u                                                21

<210> 23
<211> 21
<212> RNA
<213> Artificial Sequence

<400> 23
aagcucaccu gaggacucat t                                                21

<210> 24
<211> 21
<212> RNA
<213> Artificial Sequence

<400> 24
ugaguccuca ggugagcuut t                                                21

<210> 25
<211> 19
<212> RNA
<213> Artificial Sequence

<400> 25
gcaucaacau gaacgucuu                                                   19

<210> 26
<211> 21
<212> RNA
<213> Artificial Sequence

<400> 26
aagacguuca uguugaugcu g                                                21

<210> 27
<211> 21
<212> RNA
<213> Artificial Sequence

<400> 27
cugaguuuaa aaggcaccct t                                                21

<210> 28
<211> 21
<212> RNA
<213> Artificial Sequence

<400> 28
gggugccuuu uaaacucagt t                                                21

## Description

### TECHNICAL FIELD

The present invention relates to a composition for suppressing the expression of a target gene in an eyeball, and the like.

### BACKGROUND ART

As a method of suppressing the expression of a target gene, for example, a method utilizing RNA interference (hereinafter, referred to as RNAi) and the like are known, specifically, a phenomenon in which when a double-stranded RNA having the same sequence as that of a target gene is introduced into Nematode, the expression of the target gene is specifically suppressed has been reported (see "Nature", Vol. 391, No. 6669, pp.806-811, 1998). In addition, it has been found that when a double-stranded RNA having a length of 21 to 23 nucleotides is introduced into Drosophila, in place of a long double-stranded RNA, the expression of a target gene is suppressed, and this is named short interfering RNA (siRNA) (see International Publication No. WO 01/75164).

In the case of mammalian cells, when a long double-stranded RNA was introduced, apoptosis occurred by the functions of virus defense mechanism, and the expression of a specific gene could not be suppressed, but it has been found that in the case of siRNA having a length of 20 to 29 nucleotides, such a reaction does not occur, and the expression of a specific gene can be suppressed. Inter alia, siRNA having a length of 21 to 25 nucleotides has a high effect of suppressing expression ("Nature", Vol. 411, No. 6836, pp.494-498, 2001; "Nature Reviews Genetics", Vol. 3, No. 10, pp.737-747, 2002; "Molecular Cell", USA, Vol. 10, No. 3, pp.549-561, 2002; "Nature Biotechnology", USA, Vol. 20, No. 5, pp.497-500, 2002). In addition, it has reported that not a double-stranded RNA but a single stranded RNA having a hairpin structure as a result of intermolecular hybridization exhibits RNAi like siRNA (see "Proceedings of the National Academy of Sciences of the United States of America", Vol. 99, No. 9, pp.6047-6052, 2002).

RNAi has been studied much in also in vivo tests, and the effect of RNAi using siRNA with a length of not more than 50 nucleotide pairs on fetal animals (see Patent Document 1) and the effect on adult mice (see Patent Document 2) have been reported. In addition, when siRNA is intravenously administered to a fetal mouse, the effect of suppressing the expression of a specific gene has been found in various organs such as kidney, spleen, lung, pancreas, and liver (see Non-Patent Document 1). Further, it has been reported that when siRNA is directly administered to brain cells, the expression of a specific gene is also suppressed (see Non-Patent Document 2).

On the other hand, diseases in eyeball, such as diabetic retinopathy, retinopathy of prematurity, age related macular degeneration and the like, are main factors for blindness in USA and the world, and a biochemical event regarding these processes has not been completely elucidated.

Diabetic retinopathy occurs as a result of damage of a retina. A retina has a role in sensing light or a color and transmitting it to a brain, wherein numerous fine blood vessels (micro blood vessels) are networked. In the case of diabetes, much glucose is contained in blood, a blood vessel wall is damaged, and a micro blood vessel is obstructed, resulting in microangiopathy. For this reason, oxygen and a nutrient in a retina are lacked, and then a pathologic neovessel appears, resulting in retinopathy exhibiting a symptom such as fundal hemorrhage, vitreous bleeding and the like.

Retinopathy of prematurity is hemorrhage and detachment of a retina occurring in a low birth weight infant. In the case of a low birth weight infant, a blood vessel of a retina is not sometimes sufficiently developed, oxygen administration immediately after birth motivates to generate a pathologic angiogenesis, resulting in retinal detachment and reduced eyesight.

Age related macular degeneration is a disease in which a pathologic neovessel is generated and then hemorrhage occurs, and a tissue of a retina is damaged on macula situated at a center of fundus oculi and most influencing on eyesight. A main factor of the disease is thought to be aging, but a cause has not been made clear. With aging of society, a number of patients are increased, in U.S.A., age related macular degeneration is at a top of a cause for blindness of an elderly.

Currently, targeting age related macular degeneration, a clinical trial of siRNA medicine is being progressed, but in any case, a trial is conducted by intraocular administration, and invasiveness into a body is high.

That is, for the purpose of suppressing the expression of a target gene in an eyeball, development of a method of effectively delivering RNA suppressing the expression of the target gene to a target tissue by systemic administration in place of administration into an eyeball is required.

On the other hand, as means for delivering a nucleic acid into a cell, a method of using a cationic liposome or cationic polymer is known. However, by the method, after intravenous administration of cationic liposome or cationic polymers comprising a nucleic acid is carried out, the nucleic acid is promptly removed from the blood, and when a target tissue is different from liver or lung, for example, when it is a tumor site or the like, the nucleic acid cannot be delivered to the target tissue, and therefore, the expression of a sufficient action has not been made possible yet. Accordingly, a nucleic acid-encapsulating liposome (liposome encapsulating a nucleic acid therein) with which the problem that a nucleic acid is promptly removed from the blood was solved has been reported (see Patent documents 3 to 5, and Non-patent document 3). In the Patent document 3, as a method of producing liposome comprising a nucleic acid or the like, for example, a method of producing an ODN-encapsulating liposome by dissolving a cationic lipid in chloroform in advance, adding an aqueous solution of oligodeoxynucleotide (ODN) and methanol thereto and mixing and centrifuging the mixture thereby transferring a complex of the cationic lipid and ODN to a chloroform layer, and then taking out the chloroform layer, adding a polyethylene glycolated phospholipid, a neutral lipid and water to the chloroform layer to form a water-in-oil (w/o) emulsion and treating the emulsion by the reverse phase evaporation method has been reported. In the Patent document 4 and the Non-patent document 3, a method of producing an ODN-encapsulating liposome by dissolving ODN in an aqueous solution of citric acid at pH 3.8, adding a lipid (in ethanol) to the solution, reducing the ethanol concentration to 20 v/v% to prepare an ODN-encapsulating liposome, performing filtration for sizing, removing excess ethanol by dialysis, and then further performing dialysis of the sample at pH 7.5 to remove ODN adhering to the surface of the liposome has been reported. In each method, liposome encapsulating an active ingredient such as a nucleic acid is produced.

On the other hand, in the Patent document 5, it has been reported that liposome encapsulating an active ingredient such as a nucleic acid is produced by a method of coating fine particles with a bilayer lipid membrane in a liquid. In the method, fine particles are coated with a bilayer lipid membrane by reducing the ratio of a polar organic solvent in an aqueous solution comprising the polar organic solvent in which the fine particles are dispersed and a lipid is dissolved. The coating is carried out in the liquid, and for example, fine particles coated with a bilayer lipid membrane (coated fine particles) having a size suitable for fine particles for intravenous injection and the like are produced very efficiently. In addition, as an example of the fine particles, for example, a complex which comprises a water-soluble drug and a cationic lipid and formed by an electrostatic interaction is exemplified in the Patent document 5. It has been reported that the particle diameter of coated fine particles obtained by coating the complex particles varies depending on the complex particles to be coated, however, the coated fine particles obtained by coating the ODN-lipid complex have a small particle diameter and can be used as an injection, and the coated complex particles show a high retention in the blood and are much accumulated in a tumor tissue when they are intravenously administered.
Patent Document 1: International Publication No. WO 02/132788
Patent Document 2: International Publication No. WO 03/10180
Patent Document 3: Published Japanese Translation of PCT International Application No.2002-508765
Patent Document 4: Published Japanese Translation of PCT International Application No.2002-501511
Patent Document 5: International Publication No. WO 02/28367
Non-Patent Document 1: Nature Genetics, Vol. 32, No. 1, pp.107-108, 2002
Non-Patent Document 2: Nature Biotechnology, Vol. 20, No. 10, pp.1006-1010, 2002
Non-Patent Document 3: Biochimica et Biophysica Acta, Vol. 1510, pp.152-166, 2001

### DISCLOSURE OF THE INVENTION

### PROBLEMS TO BE SOLVED BY THE INVENTION

An object of the present invention is to provide a composition for suppressing the expression of a target gene in an eyeball, a medicament for treating of a disease in an eyeball, and the like.

### MEANS FOR SOLVING THE PROBLEMS

The present invention relates to the following (1) to (54).
(1) A composition comprising an RNA-encapsulated liposome, the RNA comprising a sequence being of 15 to 30 contiguous nucleotides of mRNA of a target gene associated with a disease in an eyeball, and a sequence complementary to the sequence,
   wherein the liposome is capable of reaching an eyeball.
(2) The composition according to (1), wherein the liposome is a liposome having a size that allows intravenous administration thereof.
(3) The composition according to (1) or (2), wherein the RNA is RNA having an action of suppressing the expression of the target gene utilizing RNA interference (RNAi).
(4) The composition according to any one of (1) to (3), wherein the target gene associated with a disease in an eyeball is a gene involved in angiogenesis in an eye disease.
(5) The composition according to any one of (1) to (3), wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.
(6) The composition according to any one of (1) to (3), wherein the mRNA of a target gene associated with a disease in an eyeball is KLF5 mRNA.
(7) The composition according to any one of (1) to (6), wherein the mRNA is either human or mouse mRNA.
(8) The composition according to any one of (1) to (7), wherein the RNA-encapsulated liposome is a liposome composing a complex particle comprising a lead particle and the RNA as constituent components; and a bilayer lipid membrane for coating the complex particle; and
   wherein the constituent components of the bilayer lipid membrane are soluble in a polar organic solvent, and the constituent components of the bilayer lipid membrane and the complex particle are dispersible in a liquid containing the polar organic solvent at a specific concentration.
(9) The composition according to (8), wherein the polar organic solvent is an alcohol.
(10) The composition according to (8), wherein the polar organic solvent is ethanol.
(11) The composition according to any one of (8) to (10), wherein the lead particle is a lead particle comprising a cationic lipid, and
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(12) The composition according to any one of (1) to (7), wherein the RNA-encapsulated liposome is a liposome composing a complex particle comprising a lead particle and the RNA as constituent components, the lead particle comprising a cationic lipid; and a bilayer lipid membrane for coating the complex particle; and
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(13) The composition according to (11) or (12), wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.
(14) The composition according to any one of (11) to (13), wherein the lipid derivative, the fatty acid derivative or the aliphatic hydrocarbon derivative of a water-soluble substance is polyethylene glycol phosphatidyl ethanolamine.
(15) The composition according to any one of (11) to (14), wherein the neutral lipid is egg yolk phosphatidylcholine.
(16) A medicament for treating of a disease in an eyeball comprising an RNA-encapsulated liposome, the RNA-encapsulated liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
   wherein the constituent components of the bilayer lipid membrane are soluble in a polar organic solvent, and the constituent components of the bilayer lipid membrane and the complex particle are dispersible in a liquid comprising the polar organic solvent at a specific concentration.
(17) The medicament for treating of a disease in an eyeball according to (16), wherein the polar organic solvent is an alcohol.
(18) The medicament for treating of a disease in an eyeball according to (16), wherein the polar organic solvent is ethanol.
(19) The medicament for treating of a disease in an eyeball according to any one of (16) to (18), wherein the lead particle is a lead particle comprising a cationic lipid, and
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(20) A medicament for treating of a disease in an eyeball comprising an RNA-encapsulated liposome, the RNA-encapsulated liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(21) The medicament for treating of a disease in an eyeball according to (19) or (20), wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.
(22) The medicament for treating of a disease in an eyeball according to any one of (19) to (21), wherein the lipid derivative, the fatty acid derivative or the aliphatic hydrocarbon derivative of a water-soluble substance is polyethylene glycol phosphatidyl ethanolamine.
(23) The medicament for treating of a disease in an eyeball according to any one of (19) to (22), wherein the neutral lipid is egg yolk phosphatidylcholine.
(24) The medicament for treating of a disease in an eyeball according to any one of (16) to (23), wherein the RNA is RNA having an action of suppressing the expression of the target gene utilizing RNA interference (RNAi).
(25) The medicament for treating of a disease in an eyeball according to any one of (16) to (23), wherein the target gene associated with a disease in an eyeball is a gene involved in angiogenesis in an eye disease.
(26) The medicament for treating of a disease in an eyeball according to any one of (16) to (23), wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.
(27) The medicament for treating of a disease in an eyeball according to any one of (16) to (23), wherein the mRNA of a target gene associated with a disease in an eyeball is KLF5 mRNA.
(28) The medicament for treating of a disease in an eyeball according to any one of (16) to (27), wherein the mRNA is either human or mouse mRNA.
(29) A method of treating a disease in an eyeball, comprising administering a composition comprising an RNA-encapsulated liposome to a mammal, the liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
   wherein the constituent components of the bilayer lipid membrane are soluble in a polar organic solvent, and
   wherein the constituent components of the bilayer lipid membrane and the complex particle are dispersible in a liquid comprising the polar organic solvent at a specific concentration.
(30) The method of treating a disease in an eyeball according to (29), wherein the polar organic solvent is an alcohol.
(31) The method of treating a disease in an eyeball according to (29), wherein the polar organic solvent is ethanol.
(32) The method of treating a disease in an eyeball according to any one of (29) to (31), wherein the lead particle is a lead particle comprising a cationic lipid, and
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(33) A method of treating a disease in an eyeball, comprising administering a composition comprising an RNA-encapsulated liposome to a mammal, the liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(34) The method of treating a disease in an eyeball according to (32) or (33), wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.
(35) The method of treating a disease in an eyeball according to any one of (32) to (34), wherein the lipid derivative, the fatty acid derivative or the aliphatic hydrocarbon derivative of a water-soluble substance is polyethylene glycol phosphatidyl ethanolamine.
(36) The method of treating a disease in an eyeball according to any one of (32) to (35), wherein the neutral lipid is egg yolk phosphatidylcholine.
(37) The method of treating a disease in an eyeball according to any one of (29) to (36), wherein the RNA is RNA having an action of suppressing the expression of the target gene utilizing RNA interference (RNAi).
(38) The method of treating a disease in an eyeball according to any one of (29) to (36), wherein the target gene associated with a disease in an eyeball is a gene involved in angiogenesis in an eye disease.
(39) The method of treating a disease in an eyeball according to any one of (29) to (36), wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.
(40) The method of treating a disease in an eyeball according to any one of (29) to (36), wherein the mRNA of a target gene associated with a disease in an eyeball is KLF5 mRNA.
(41) The method of treating a disease in an eyeball according to any one of (29) to (40), wherein the mRNA is either human or mouse mRNA.
(42) Use of a composition comprising an RNA-encapsulated liposome for production of a medicament for treating of a disease in an eyeball, the liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
   wherein the constituent components of the bilayer lipid membrane are soluble in a polar organic solvent, and
   wherein the constituent components of the bilayer lipid membrane and the complex particle are dispersible in a liquid comprising the polar organic solvent at a specific concentration.
(43) The use according to (42), wherein the polar organic solvent is an alcohol.
(44) The use according to (42), wherein the polar organic solvent is ethanol.
(45) The use according to any one of (42) to (44), wherein the lead particle is a lead particle comprising a cationic lipid, and
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(46) Use of a composition comprising an RNA-encapsulated liposome for production of a medicament for treating of a disease in an eyeball, the liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
   wherein the bilayer lipid membrane is a bilayer lipid membrane comprising a neutral lipid, and a lipid derivative, a fatty acid derivative or an aliphatic hydrocarbon derivative of a water-soluble substance as constituent components.
(47) The use according to (45) or (46), wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.
(48) The use according to any one of (45) to (47), wherein the lipid derivative, the fatty acid derivative or the aliphatic hydrocarbon derivative of a water-soluble substance is polyethylene glycol phosphatidyl ethanolamine.
(49) The use according to any one of (45) to (48), wherein the neutral lipid is egg yolk phosphatidylcholine.
(50) The use according to any one of (42) to (49), wherein the RNA is RNA having an action of suppressing the expression of the target gene utilizing RNA interference (RNAi).
(51) The use according to any one of (42) to (49), wherein the target gene associated with a disease in an eyeball is a gene involved in angiogenesis in an eye disease.
(52) The use according to any one of (42) to (49), wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.
(53) The use according to any one of (42) to (49), wherein the mRNA of a target gene associated with a disease in an eyeball is KLF5 mRNA.
(54) The use according to any one of (42) to (53), wherein the mRNA is either human or mouse mRNA.

### EFFECTS OF THE INVENTION

By administering a composition comprising an RNA-encapsulated liposome, RNA comprising a sequence being of 15 to 30 contiguous nucleotides of mRNA of a target gene associated with a disease in an eyeball, and a sequence complementary to the sequence according to the present invention, to a mammal or the like, the expression of the target gene associated with a disease in an eyeball can be suppressed. In addition, by suppressing the expression of the target gene associated with a disease in an eyeball, a disease in an eyeball can be treated.

### BRIEF DESCRIPTION OF THE DRAWINGS

Fig. 1 is a view showing a fundus fluorescent angiogram after 2 hours following administration of the preparation obtained in Example 1.
Fig. 2 is a view showing a fundus fluorescent angiogram after 8 hours following administration of the preparation obtained in Example 1.
Fig. 3 is a view showing a fundus fluorescent angiogram after 168 hours following administration of the preparation obtained in Example 1.
Fig. 4 is a view showing a change in a sum of an intensity of each pixel of a lesion to which the preparation obtained in Example 1 was administered.
Fig. 5 is a view showing fluorescence derived from FITC in a retina section after 2 hours following administration of the preparation obtained in Example 1.
Fig. 6 is a view showing fluorescence derived from FITC in a retina section after 4 hours following administration of the preparation obtained in Example 1.
Fig. 7 is a view showing fluorescence derived from FITC in a retina section after 168 hours following administration of the preparation obtained in Example 1.
Fig. 8 is a view showing fluorescence derived from FAM in a retina section after 2 hours following administration of the preparation obtained in Example 1.
Fig. 9 is a view showing fluorescence derived from FAM in a retina section after 4 hours following administration of the preparation obtained in Example 1.
Fig. 10 is a view showing fluorescence derived from FAM in a retina section after 168 hours following administration of the preparation obtained in Example 1.
Fig. 11 is a view showing choroidal neovascularization after 72 hours following primary administration with administering a preparation obtained in Example 2 three times every 24 hours.
Fig.12 is a view showing choroidal neovascularization after 72 hours following primary administration with administering a preparation obtained in Example 3 three times every 24 hours.

### BEST MODE FOR CARRYING OUT THE INVENTION

The target gene associated with a diseases in an eyeball in the present invention is not particularly limited as long as it is an eye disease-associated gene which is expressed by producing mRNA in an eyeball of a mammal, but as the eye disease-associated gene, for example, a gene involved in formation of the pathologic conditions of an eye disease is preferable, a gene involved in angiogenesis or the like in an eye disease is more preferable, examples thereof include genes or the like encoding a protein such as vascular endothelial growth factor (hereinafter abbreviated as VEGF), vascular endothelial growth factor receptor (hereinafter abbreviated as VEGFR), fibroblast growth factor, fibroblast growth factor receptor, platelet-derived growth factor, platelet-derived growth factor receptor, hepatocyte growth factor, hepatocyte growth factor receptor, Kruppel-like factor (hereinafter, abbreviated as KLF), Ets transcription factor, nuclear factor, hypoxia-inducible factor and the like, specifically, VEGF gene, VEGFR gene, fibroblast growth factor gene, fibroblast growth factor receptor gene, platelet-derived growth factor gene, platelet-derived growth factor receptor gene, hepatocyte growth factor gene, hepatocyte growth factor receptor gene, KLF gene, Ets transcription factor gene, nuclear factor gene, hypoxia-inducible factor gene and the like, preferably VEGF gene, VEGFR gene, KLF gene and the like, more preferably KLF gene, still more preferably KLF5 gene.

KLF includes a KLF family. The family is a family of transcription factors, which is characterized in that it has a zinc finger motif at the C-terminus thereof, and includes KLF1, KLF2, KLF3, KLF4, KLF5, KLF6, KLF7, KLF8, KLF9, KLF10, KLF11, KLF12, KLF13, KLF14, KLF15, KLF16 and the like. It has been reported that, in mammals, the KLF family is important in differentiation of various types of tissues or cells, such as erythrocyte, vascular endothelial cell, smooth muscle, skin, lymphocyte and the like, and plays an important role in formation of the pathologic conditions of various types of diseases such as cancer, cardiovascular disease, cirrhosis hepatic, renal disease and immune-mediated disease (The Journal of Biological Chemistry Vol. 276, No. 37, pp.34355-34358, 2001, Genome Biology, Vol. 4, No. 2, p.206, 2003).

Among the KLF family members, KLF5 is also referred to as BTEB2 (basic transcriptional element binding protein 2) or IKLF (intestinal-enriched Kruppel-like factor). The expression of KLF5 in vascular smooth muscle is controlled at the development stage thereof. KLF5 is highly expressed in the vascular smooth muscle of a fetus, whereas its expression is not found in the vascular smooth muscle of a healthy adult. In addition, in the case of the smooth muscle of intima of a blood vessel regenerated after denudation by a balloon catheter, KLF5 is highly expressed. Also, in the smooth muscle in lesions due to arteriosclerosis or restenosis, KLF5 is expressed (Circulation, Vol. 102, No. 20, pp. 2528-2534, 2000).

VEGF is a growth factor specific for a vascular endothelial cell found by Ferrara et al., 1983. In the same year, a factor having the vascular permeating activity was found by Senger, Dvorak et al., and named VPF (vascular permeability factor). As a result of amino acid sequence analysis of a protein, it has found that two are same. VEGF binds to a receptor of an endothelial cell inside a blood vessel to promote growth. VEGF not only produces a blood vessel at a fetal stage, but also acts when a pathological blood vessel is generated. For example, when a cancer is grown to some extent and becomes oxygen-deficient state, VEGF and a receptor thereof are grown to cause angiogenesis. In addition, the vascular permeation exacerbation is considered to be a cause for cancerous ascites. When diabetes progresses, a new blood vessel is generated in a retina, and this is acted by VEGF. That is, this is a protein producing a new blood vessel. It can be said that it has an important role in angiogenesis by inducement of its expression by the hypoxia state. For explaining a mechanism of edema found in a tumor, an inflammatory lesion or the like as well as angiogenesis, involvement of this factor is strongly suggested.

On the other hand, VEGFR is possessed by a vascular endothelial cell and a cancer cell themselves and, by binding of these substances to a receptor thereof, the receptor itself is phosphorylated (activated) and, as a result, various directions such as proliferation and migration are transmitted. It is known that, by inhibiting phosphorylation of this receptor, transmission in a cell is inhibited, and angiogenesis is inhibited.

Examples of the RNA in the present invention include an RNA comprising a sequence being of 15 to 30, preferably 17 to 25, and more preferably 19 to 23 contiguous nucleotides of the above-mentioned target gene mRNA and a sequence complementary to the sequence. The RNA in the present invention includes a derivative in which an oxygen atom or the like contained in a phosphate moiety, an ester moiety or the like in the ribonucleic acid structure has been substituted with another atom such as a sulfur atom are included. Further, preferred examples of the RNA in the present invention include an RNA having an action of suppressing the expression of the target gene utilizing RNA interference (RNAi). Here, by taking an RNA capable of suppressing the expression of KLF5 gene as an example, the RNA capable of suppressing the expression of the target gene by utilizing RNA interference (RNAi) will be explained. Other genes also have a similar structure and can be obtained by a similar procedure.

RNA capable of suppressing the expression of KLF5 gene contains a sequence being of 15 to 30, preferably 17 to 25, and more preferably 19 to 23 contiguous nucleotides of KLF5 mRNA (hereinafter referred to as sequence X) and a sequence complementary to the sequence (hereinafter referred to as complementary sequence X'). Examples of such an RNA include: (A) an RNA which is a double-stranded RNA having a strand of sequence X (sense strand) and a strand of complementary sequence X' (antisense strand), in which 1 to 6, and preferably 2 to 4 nucleotides are added to the 3'-terminus of the strand of the sequence X or the strand of the complementary sequence X',or each of the strands in the same manner or different manner, and which suppresses the expression of KLF5 gene (hereinafter, RNA having such a structure is referred to as KLF5 siRNA), and (B) an RNA, which has a hairpin structure, and in which an RNA having sequence X is ligated to an RNA having complementary sequence X' via a spacer oligonucleotide, and 1 to 6, and preferably 2 to 4 nucleotides are added to the 3'-terminus thereof, and which suppressed the expression of KLF5 gene (hereinafter, such RNA is referred to as KLF5 shRNA) and the like. The bases of the nucleotides to be added to such an RNA may be the same or different, and are independently selected from guanine, adenine, cytosine, thymine and uracil, and either RNA or DNA may be used as the nucleotide, however, either one member or two members selected from uridylic acid (U) and deoxythymidylic acid (dT) is/are preferred. As the spacer oligonucleotide, an RNA consisting of 6 to 12 nucleotides is preferred. As the sequence at the 5'-terminus thereof, two nucleotides, UU, are preferred. Examples of the spacer oligonucleotide include an RNA consisting of the sequence UUCAAGAGA. Either of the two RNA portions that are ligated to each other via the spacer oligonucleotide may be suitable as the RNA on the 5'-terminal side. In addition, in any case, a sequence of a base of a nucleotide added adjacent to the 3'-terminal side of the complementary sequence X' may be a sequence complementary to a sequence of a base adjacent to the sequence X in a mRNA, and this structure is more preferable. The sequence X may be any sequence as long as it is a sequence being of 15 to 30, preferably 17 to 25, and more preferably 19 to 23 contiguous nucleotides of KLF5 mRNA, however, a sequence being of 19 nucleotides designed by the method described in International Publication No. WO 2005/010185 is most preferable.

Examples of RNA capable of suppressing the expression of KLF5 gene include RNAs shown in Table 1, which comprise a sequence of 15 to 30 contiguous nucleotides of mouse KLF5 and a sequence complementary to the sequence. In addition, for KLF5siRNA Nos. 2 to 4, and 7 to 11 in Table 1, human sequences corresponding to sequences selected in a mouse can be obtained by aligning mouse KLF5 cDNA and human KLF5 cDNA based on homology of sequences.

**(Table 1)**

| KLF5si RNA No. | Sequence of RNA | SEQ ID No. |
|---|---|---|
| No. 1 | CAUGAACGUCUUCCUCCCUTT | SEQ ID No.: 1 |
| | AGGGAGGAAGACGUUCAUGTT | SEQ ID No.: 2 |
| No. 2 | AUUUACCUGCCACUCUGCCUU | SEQ ID No.: 3 |
| | GGCAGAGUGGCAGGUAAAUUU | SEQ ID No.: 4 |
| No. 3 | GGAGUAACCCGGAUCUGGAUU | SEQ ID No.: 5 |
| | UCCAGAUCCGGGUUACUCCUU | SEQ ID No.: 6 |
| No. 4 | AAGCUCACCUGAGGACUCAUU | SEQ ID No.: 7 |
| | UGAGUCCUCAGGUGAGCUUUU | SEQ ID No.: 8 |
| No. 5 | UCCCCAGACCGUCCAUGCCUU | SEQ ID No.: 9 |
| | GGCAUGGACGGUCUGGGGGUU | SEQ ID No.: 10 |
| No. 6 | CGCUGCGCCCACCCGCCUGUU | SEQ ID No.: 11 |
| | CAGGCGGGUGGGCGCAGCGUU | SEQ ID No.: 12 |
| No. 7 | AUGGAGAAGUAUCUGACCCUU | SEQ ID No.: 13 |
| | GGGUCAGAUACUUCUCCAUUU | SEQ ID No.: 14 |
| No. 8 | AGUAUAGACGAGACAGUGCUU | SEQ ID No.: 15 |
| | GCACUGUCUCGUCUAUACUUU | SEQ ID No.: 16 |
| No. 9 | ACCAGACGGCAGUAAUGGAUU | SEQ ID No.: 17 |
| | UCCAUUACUGCCGUCUGGCUU | SEQ ID No.: 18 |
| No. 10 | GCUCAGAGCCUGGAAGUCCUU | SEQ ID No.: 19 |
| | GGACUUCCAGGCUCUGAGCUU | SEQ ID No.: 20 |
| No. 11 | GCCGUUCCAGUGCAUGGUGUU | SEQ ID No.: 21 |
| | CACCAUGCACUGGAACGGCUU | SEQ ID No.: 22 |

In addition, KLF5siRNA No.1 in Table 1 can be prepared by chemical synthesis and annealing by requesting, for example, Japan Bio service Co., Ltd. KLF5siRNA No.2 to 11 in Table 1 can be prepared by in vitro transcription utilizing Silencer siRNA manufacturing kit (Silencer (registered trade mark) siRNA Construction Kit, manufactured by Ambion Ltd.). A DNA used in manufacturing a template of in vitro transcription can be obtained by requesting, for example, Hokkaido System Science Co., Ltd. to chemically synthesize it.

As a liposome in the composition of the present invention (hereinafter, referred to as liposome A), an RNA-encapsulated liposome which encapsulates an RNA comprising a sequence being of 15 to 30 contiguous nucleotides of mRNA of a target gene associated with a disease in an eyeball, and a sequence complementary to the sequence is preferable. The liposome A is not particularly limited as long as it is capable of reaching a tissue or an organ including an expression site of the target gene, but examples include a liposome which comprises a complex particle comprising a lead particle and the RNA as constituent components, and a bilayer lipid membrane for coating the complex particle and the like, and it is preferable that constituent components of the bilayer lipid membrane are soluble in a polar organic solvent, and the constituent components of the bilayer lipid membrane and the complex particles are dispersible in a liquid containing the polar organic solvent at a specific concentration. In addition, examples of the liposome A also include a liposome which comprises a complex particle comprising a lead particle and the RNA as constituent components, the lead particle comprising a cationic lipid; and a bilayer lipid membrane for coating the complex particle; in which the bilayer lipid membrane contains, as constituent components, a neutral lipid and a lipid derivative, a fatty acid derivative or an aliphatic hydrocarbon derivative, of a water-soluble substance, and it is more preferable that constituent components of the bilayer lipid membrane are soluble in a polar organic solvent, and the constituent components of the bilayer lipid membrane and the complex particles are dispersible in a liquid containing the polar organic solvent at a specific concentration.

In the present invention, "dispersing" means "dispersing without dissolution".

Examples of the lead particle in the present invention include fine particles comprising, as a constituent component, for example, a lipid assembly, liposome, an emulsion particle, a polymer, a metal colloid, a fine particle preparation or the like. Preferred examples thereof include a fine particle comprising liposome as a constituent component. The lead particle in the present invention may contain, as a constituent component, a complex obtained by combining two or more members selected from a lipid assembly, liposome, an emulsion particle, a polymer, a metal colloid, a fine particle preparation and the like, or may contain, as a constituent component, a complex obtained by combining a lipid assembly, liposome, an emulsion particle, a polymer, a metal colloid, a fine particle preparation or the like with another compound (such as a sugar, a lipid or an inorganic compound), and the like.

The lipid aggregate or the liposome (hereinafter, referred to as liposome B) as a constituent component of the lead particle is preferably constituted of, for example, a lipid and/or a surfactant. The lipid may be any of simple lipid, complex lipid and derived lipid, and examples thereof include phospholipid, neutral lipid, glyceroglycolipid, sphingoglycolipid, sphingoid, sterol, cationic lipid and the like, but not limited to. Preferable examples thereof include a phospholipid and a cationic lipid. In addition, the lipid also includes a lipid derivative such as a surfactant (having the same meaning as that of a surfactant described later), a polymer (having the same meaning as that of a polymer described later, specifically dextran etc.) and polyoxyethylene derivative (specifically, polyethylene glycol etc.). Examples of the surfactant include a nonionic surfactant, an anionic surfactant, a cationic surfactant, an amphoteric surfactant and the like.

Examples of the phospholipid in a lipid constituting the lead particle include natural or synthetic phospholipids such as phosphatidylcholine (specifically, soybean phosphatidylcholine, egg yolk phosphatidylcholine (EPC), distearoylphosphatidylcholine, dipalmitoylphosphatidylcholine, dimyristoylphosphatidylcholine, dioleoylphosphatidylcholine, etc.), phosphatidylethanolamine (specifically, distearoylphosphatidylethanolamine (DSPE), dipalmitoylphosphatidylethanolamine, dioleoylphosphatidylethanolamine etc.), glycerophospholipid (specifically, phosphatidylserine, phosphatidic acid, phosphatidylglycerol, phosphatidylinositol, lysophosphatidylcholine etc.), sphingophospholipid (specifically, sphingomyelin, ceramide phosphoethanolamine, ceramide phosphoglycerol, ceramide phosphoglycerophosphoric acid etc.), glycerophosphonolipid, sphingophosphonolipid, natural lecithin (specifically egg yolk lecithin, soybean lecithin etc.), hydrogenated phospholipid (specifically, hydrogenated soybean phosphatidylcholine etc.) and the like.

Examples of the glyceroglycolipid in a lipid constituting the lead particle include sulfoxyribosylglyceride, diglycosyldiglyceride, digalactosyldiglyceride, galactosyldiglyceride, glycosyldiglyceride and the like.

Examples of the sphingoglycolipid in a lipid constituting the lead particle include galactosylcerebroside, lactosylcerebroside, ganglioside and the like.

Examples of sphingoid in a lipid constituting the lead particle include sphingan, icosasphingan, sphingosine and derivatives thereof. Examples of the derivatives include sphingan, icosasphingan and sphingosine, in which -NH₂ is converted into -NHCO(CH₂)ₓCH₃ (wherein x represents an integer of 0 to 18 and, inter alia, preferably 6, 12 or 18), and the like.

Examples of sterol in a lipid constituting the lead particle include cholesterol, dihydrocholesterol, lanosterol, β-sitosterol, kampesterol, stigmasterol, brassicasterol, ergocasterol, fucosterol, 3 β-[N-(N',N'-dimethylaminoethyl) carbamoyl]cholesterol (DC-Chol) and the like.

Examples of the cationic lipid in a lipid constituting the lead particle include N-[1-(2,3-dioleoylpropyl)]-N,N,N-trimethyl ammonium chloride (DOTAP), N-[1-(2,3-dioleoylpropyl)]-N,N-dimethylamine (DODAP), N-[1-(2,3-dioleyloxypropyl)]-N,N,N-trimethyl ammonium chloride (DOTMA), 2,3-dioleyloxy-N-[2-(sperminecarboxyamido)ethyl]-N,N-dimeth yl-1-propanaminiumtrifluoroacetic acid (DOSPA), N-[1-(2,3-ditetradecyloxypropyl)]-N,N-dimethyl-N-hydroxyethyl ammonium bromide (DMRIE), N-[1-(2,3- dioleyloxypropyl)]-N,N-dimethyl-N-hydroxyethyl ammonium bromide (DORIE) and the like.

Examples of the nonionic surfactant constituting the lead particle include monooleic acid polyoxyethylene sorbitan (specifically, Polysorbate 80 etc.), polyoxyethylene polyoxypropylene glycol (specifically, Pluronic F68 etc.), sorbitan fatty acid ester (specifically, sorbitan monolaurate, sorbitan monooleate etc.), a polyoxyethylene derivative (specifically, polyoxyethylene hardened castor oil 60, polyoxyethylene lauryl alcohol etc.), glycerin fatty acid ester, and the like.

Examples of the anionic surfactant constituting the lead particle include acylsarcosine, sodium alkylsulfate, alkylbenzenesulfonate, and sodium fatty acid having a carbon number of 7 to 22, and the like. Specifically, examples thereof include sodium dodecylsulfate, sodium laurylsulfate, sodium cholate, sodium deoxycholate, sodium taurodeoxycholate and the like.

Examples of the cationic surfactant constituting the lead particle include an alkylamine salt, an acylamine salt, a quaternary ammonium salt, an amine derivative and the like. Specifically, examples thereof include benzalkonium chloride, an acylaminoethyldiethylamine salt, an N-alkylpolyalkylpolyamine salt, fatty acid polyethylene polyamide, cetyltrimethylammonium bromide, dodecyltrimethylammonium bromide, alkylpolyoxyethyleneamine, N-alkylaminopropylamine, fatty acid triethanolamine ester and the like.

Examples of the amphoteric surfactant constituting the lead particle include 3-[(3-colamidopropyl) dimethylammonio]-1-propanesulfonic acid, N-tetradecyl-N,N-dimethyl-3-ammonio-1-propanesulfonic acid and the like.

In the liposome B, these lipids and surfactants may be used alone, or may be used by combining two or more kinds, preferably, are used by combining two or more kinds. Examples of a combination when two or more kinds are used by combination include a combination of two or more components selected from hydrogenated soybean phosphatidylcholine, polyethylene glycolated phospholipid and cholesterol, a combination of two or more components selected from distearoylphosphatidylcholine, polyethylene glycolated phospholipid and cholesterol, a combination of EPC and DOTAP, a combination of EPC, DOTAP and polyethylene glycolated phospholipid, and a combination of EPC, DOTAP, cholesterol and polyethylene glycolated phospholipid, and the like.

In addition, the liposome B may optionally contain a membrane stabilizer such as sterol such as cholesterol, or an antioxidant such as tocopherol. These stabilizers may be used alone, or may be used by combining two or more kinds.

Examples of the lipid aggregate include a spherical micelle, a spherical reverse micelle, a sausage-like micelle, a sausage-like reverse micelle, a plate-like micelle, a plate-like reverse micelle, hexagonal I, hexagonal II and an associate comprising two or more molecules of lipid, and the like.

Examples of the emulsion particle include oil-in-water (O/W) emulsion particles and water-in-oil-in-water (W/O/W) emulsion particles such as an emulsion consisting of a fatty emulsifying agent, a nonionic surfactant and a soybean oil, a lipid emulsion, a lipid nanosphere and the like.

Examples of the polymer include natural polymers such as albumin, dextran, chitosan, dextran sulfate and DNA, synthetic polymers such as poly-L-lysine, polyethyleneimine, polyaspartic acid, styrene maleic acid copolymer, isopropylacrylamide-acrylpyrrolidone copolymer, polyethylene glycol-modified dendrimer, polylactic acid, polylactic acid polyglycolic acid and polyethylene glycolated polylactic acid, and salts thereof.

Herein, examples of a salt in the polymer include a metal salt, an ammonium salt, an acid addition salt, an organic amine addition salt, an amino acid addition salt and the like. Examples of the metal salt include an alkali metal salt such as a lithium salt, a sodium salt, a potassium salt, and the like, an alkaline earth metal salt such as a magnesium salt, a calcium salt and the like, an aluminum salt and a zinc salt. Examples of the ammonium salt include salts of ammonium and tetramethylammonium, and the like. Examples of the acid addition salt include inorganic acid salts such as hydrochloride, sulfate, nitrate and phosphate, and organic acid salts such as acetate, maleate, fumarate and citrate, and the like. Examples of the organic amine addition salt include addition salts of morpholine, piperidine and the like. Examples of the amino acid addition salt include addition salts of glycine, phenylalanine, aspartic acid, glutamic acid, lysine and the like.

Examples of the metal colloid include metal colloids comprising gold, silver, platinum, rhodium, silica, calcium, aluminum, iron, indium, cadmium, barium, lead or the like.

Examples of the fine particle preparation include a microsphere, a microcapsule, a nanocrystal, a lipid nanoparticle, a polymer micelle and the like.

In addition, the lead particle in the present invention preferably contains a lipid derivative or a fatty acid derivative of one or more substance(s) selected from a sugar, a peptide, a nucleic acid and a water soluble polymer, or a surfactant. The lipid derivative or the fatty acid derivative of one or more substance(s) selected from a sugar, a peptide, a nucleic acid and a water-soluble polymer, or the surfactant may be contained as constituent components of the lead particle, or may be used by adding to constituent components of the lead particle.

Examples of the lipid derivative or the fatty acid derivative of one or more substance(s) selected from a sugar, a peptide, a nucleic acid and a water-soluble polymer, or the surfactant include preferably a glycolipid, and a lipid derivative or a fatty acid derivative of a water-soluble polymer, more preferably a lipid derivative or a fatty acid derivative of a water-soluble polymer. The lipid derivative or the fatty acid derivative of one or more substance(s) selected from a sugar, a peptide, a nucleic acid, and a water-soluble polymer, or the surfactant is preferably a substance having two aspects, which has a nature that a part of a molecule binds to other constituent component of the lead particle with a hydrophobic affinity force, electrostatic interaction or the like, and other part binds to a solvent at the time of production of the lead particle, for example, with a hydrophilic affinity force, electrostatic interaction or the like.

Examples of the lipid derivative or the fatty acid derivative of a sugar, a peptide or a nucleic acid include derivatives in which a sugar such as sucrose, sorbitol, and lactose, a peptide such as a casein-derived peptide, an egg white-derived peptide, a soybean-derived peptide, and glutathione, or a nucleic acid such as DNA, RNA, plasmid, siRNA, and ODN is bound to a lipid listed in definition of the lead particle, or the fatty acid such as stearic acid, palmitic acid, myristic acid, and lauric acid.

In addition, the lipid derivative or the fatty acid derivative of the sugar includes, for example, glyceroglycolipid and sphingoglycolipid listed in definition of the lead particle.

Examples of the lipid derivative or the fatty acid derivative of a water-soluble polymer include those in which, for example, polyethylene glycol, polyglycerin, polyethyleneimine, polyvinyl alcohol, polyacrylic acid, polyacrylamide, oligosaccharide, dextrin, water-soluble cellulose, dextran, chondroitin sulfate, polyglycerin, chitosan, polyvinylpyrrolidone, polyaspartic acid amide, poly-L-lysine, mannan, pullulan, oligoglycerol or the derivative thereof is bound to, for example, the lipid listed in definition of the lead particle, or fatty acid such as stearic acid, palmitic acid, myristic acid, and lauric acid, more preferably a lipid derivative or the fatty acid derivative such as a polyethylene glycol derivative, a polyglycerin derivative, further preferably, a lipid derivative or a fatty acid derivative of a polyethylene glycol derivative.

Examples of the lipid derivative or the fatty acid derivative of a polyethylene glycol derivative include polyethylene glycolated lipid (specifically, polyethylene glycol phosphatidyl ethanolamine (more specifically, 1,2-distearoyl-sn-glycero-3-phosphoethanolamine-N-[methoxy( polyethylene glycol)-2000] (PEG-DSPE) etc.), polyoxyethylene hardened castor oil 60, CREMOPHOR EL etc.), polyethylene glycol sorbitan fatty acid esters (specifically, monooleic acid polyoxyethylene sorbitan etc.), polyethylene glycol fatty acid ester and the like, more preferably polyethylene glycolated lipid.

Examples of the lipid derivative or the fatty acid derivative of a polyglycerin derivative include polyglycerinated lipid (specifically, polyglycerinphosphatidylethanolamine etc.), polyglycerin fatty acid ester and the like, more preferably polyglycerinated lipid.

Examples of the surfactant include the surfactants listed in definition of the lead particle, polyethylene glycol alkyl ether and the like, preferably, polyoxyethylene polyoxypropylene glycol, glycerin fatty acid ester and polyethylene glycol alkyl ether.

The lead particle having a positive electric charge is preferable. The "positive electric charge" as used herein includes an electric charge, surface polarization and the like which generate electrostatic attraction to an electric charge in the above-mentioned RNA, intramolecular polarization and the like. In order for the lead particle to have a positive electric charge, the lead particle preferably contains a cationic substance, more preferably contains a cationic lipid.

The cationic substance to be contained in the lead particle is a substance exhibiting a cationic nature, however, even if it is an amphoteric substance having both cationic group and anionic group, the relative electronegativity varies depending on the pH, bonding to other substance or the like, therefore, the amphoteric substance can be classified into a cationic substance as the case may be. These cationic substances may be used as a constituent component of the lead particle or may be used by adding it to the constituent components of the lead particle.

Examples of the cationic substances include cationic substances among those exemplified in definition of the lead particle (specifically, cationic lipid, cationic surfactant (the same definition as above), cationic polymer etc.), a protein or a peptide which can form a complex at a pH equal to or less than an isoelectric point, and the like.

Examples of the cationic lipid include DOTAP, DODAP, DOTMA, DOSPA, DMRIE, DORIE, DC-Chol and the like.

Examples of the cationic polymer include poly-L-lysine, polyethyleneimine, polyfect, chitosan and the like.

The protein or the peptide which can form a complex at a pH equal to or less than an isoelectric point is not particularly limited as long as it is a protein or a peptide which can form a complex at a pH equal to or less than an isoelectoric point of the substance. Examples of the protein or the peptide include albumin, orosomucoid, globulin, fibrinogen, pepsin, ribonuclease T1 and the like.

The lead particle in the present invention can be produced by or in accordance with a known production method, and a lead particle produced by any production method can be used. For example, in the production of lead particle comprising, as a constituent component, liposome B, which is one type of the lead particle, a known liposome preparation method can be applied. As the known liposome preparation method, for example, liposome preparation method by Bangham, et al. (see "Journal of Molecular Biology" (J. Mol. Biol.), Vol. 13, pp. 238-252 (1965)), an ethanol injection method (see "Journal of Cell Biology" (J. Cell Biol.), Vol. 66, pp. 621-634 (1975)), a French press method (see "FEBS Letters" (FEBS Lett.), Vol. 99, pp. 210-214 (1979)), a freeze-thaw method (see "Archives of Biochemistry and Biophysics" (Arch. Biochem. Biophys.), Vol. 212, pp. 186-194 (1981)), a reverse phase evaporation method (see "Proceedings of the National Academy of Science United States of America" (Proc. Natl. Acad. Sci. USA), Vol. 75, pp. 4194-4198 (1978)), a pH gradient method (see, for example, Japanese Patent No. 2,572,554, Japanese Patent No. 2,659,136, etc.) and the like can be applied. As a solution for dispersing liposome B in the production of the liposome B, for example, water, an acid, an alkali, any of various buffers, a physiological saline solution, an amino acid infusion or the like can be used. Further, in the production of the liposome B, it is also possible to add an antioxidant such as citric acid, ascorbic acid, cysteine or ethylenediamine tetraacetic acid (EDTA), an isotonic agent such as glycerol, glucose, sodium chloride or the like. Further, the liposome can also be produced by dissolving a lipid or the like in, for example, an organic solvent such as ethanol, distilling off the solvent, adding a physiological saline solution or the like and stirring the mixture by shaking, thereby forming the liposome B.

Further, surface improvement of the lead particle such as the liposome B can be optionally carried out using, for example, a nonionic surfactants (the same definition as above), a cationic surfactants (the same definition as above), an anionic surfactants (the same definition as above), a polymer, a polyoxyethylene derivative or the like, (see "Stealth Liposomes", edited by D. D. Lasic and F. Martin, CRC Press Inc., USA, pp. 93-102 (1995)). Examples of the polymer include dextran, pullulan, mannan, amylopectin, hydroxyethylstarch and the like. Examples of the polyoxyethylene derivative include Polysorbate 80, Pluronic F68, polyoxyethylene hydrogenated castor oil 60, polyoxyethylene lauryl alcohol, PEG-DSPE and the like. The surface improvement of the liposome such as liposome B can be employed as one of the methods of incorporating lipid derivative or a fatty acid derivative of one or more substance(s) selected from sugars, peptides, nucleic acids and water-soluble polymers or a surfactant in the lead particles.

An average particle diameter of the liposome B can be freely selected upon demand, and the following lead particle diameter is preferable. Examples of a method of adjusting the average particle diameter include an extrusion method and a method in which a large multilamellar liposome vesicle (MLV) is mechanically pulverized (specifically using Manton-gaulin, a microfluidizer or the like) (see "Emulsion and Nanosuspensions for the Formulation of Poorly Soluble Drugs", edited by R. H. Muller, S. Benita and B. Bohm, Scientific Publishers, Stuttgart, Germany, pp. 267-294, 1998) and the like.

In addition, the method of producing a complex obtained by combining two or more substances selected from, for example, a lipid assembly, liposome B, an emulsion particle, a polymer, a metal colloid, a fine particle preparation and the like, which constitute the lead particle, may be, for example, a production method in which, for example, a lipid, a polymer or the like are only mixed in water. At this time, a granulation step, a sterilization step or the like can be further added as needed. Further, it is also possible to perform the formation of the complex in any of various solvents such as acetone and ether.

As for the size of the lead particle in the present invention, an average particle diameter is preferably several nanometers to several tens micrometers, more preferably 10 nm to 1000 nm, further more preferably 50 nm to 300 nm.

Examples of the constituent component of the bilayer lipid membrane for coating complex particles comprising the lead particle and RNA in the present invention include the lipids and the surfactants illustrated in the above-mentioned definition of the lead particle and the like, and preferred examples thereof include a neutral lipid among the lipids and the surfactants. Herein, the neutral lipid means a lipid excluding the cationic lipid and the cationic surfactant listed in the cationic substance when the lead particle has a positive electric charge, and the anionic lipid and the anionic surfactant listed in the adhesion-competitive agent described later among the lipids and the surfactants, and more preferable examples of the neutral lipid include phospholipid, glyceroglycolipid, a sphingoglycolipid, and the like. More preferable example includes phospholipid, and further preferable example includes EPC. These lipids and surfactants can be used alone, or can be used by combining two or more kinds.

In addition, it is preferable that the constituent components of the bilayer lipid membrane for coating complex particles are soluble in a polar organic solvent, and it is preferable that they are dispersible in a liquid containing the polar organic solvent at a specific concentration. A concentration of the polar solvent in a liquid containing the polar solvent at a specific concentration is preferably such a concentration that the constituent components of the bilayer lipid membrane are dispersible and the complex particles are also dispersible. Examples of the polar organic solvent include alcohols such as methanol, ethanol, n-propanol, 2-propanol, n-butanol, 2-butanol, and tert-butanol, glycols such as glycerin, ethylene glycol, and propylene glycol, and polyalkylene glycol such as polyethylene glycol, and the like, inter alia, an alcohol is preferable, and ethanol is more preferable.

Examples of solvents other than a polar organic solvent in a liquid containing the polar organic solvent in the present invention include water, liquid carbon dioxide, liquid hydrocarbon, carbon halide, halogenated hydrocarbon, and the like, preferably water. The solvent may contain an ion or a buffer component. The solvents can be used alone, or two or more kinds can be used and, when two or more are used, a combination of compatible solvents is preferable.

Further, examples of the lipid used in the bilayer lipid membrane for coating the complex particles include preferably a synthetic lipid. Examples of the synthetic lipid include fluorinated phosphatidylcholine, fluorinated surfactant and dialkylammonium bromide, and these may be used alone, or may be used by combining with other lipid.

Further, the bilayer lipid membrane for coating the complex particles preferably contains a lipid derivative, a fatty acid derivative or an aliphatic hydrocarbon derivative of a water-soluble substance, or the surfactant. Examples of the lipid derivative, the fatty acid derivative or the lipid hydrocarbon derivative of a water-soluble substance include a lipid derivative or a fatty acid derivative of one or more substances selected from sugar, peptide, nucleic acid and water-soluble polymer, and an aliphatic hydrocarbon derivative of one or more substance(s) selected from sugar, peptide, nucleic acid and water-soluble polymer. As the lipid derivative, the fatty acid derivative or the aliphatic hydrocarbon derivative of a water-soluble substance, the lipid derivative or the fatty acid derivative of a water-soluble polymer is more preferable, the polyethylene glycolated phospholipid is further preferable, and polyethylene glycol phosphatidyl ethanolamine is most preferable. Examples of the aliphatic hydrocarbon derivative of a water-soluble substance in the present invention include those in which the water-soluble substance is bound to, for example, an alcoholic residue of long chain aliphatic alcohol, polyoxypropylenealkyl, glycerin fatty acid ester or the like.

Examples of the aliphatic hydrocarbon derivative of a sugar, a peptide or a nucleic acid include aliphatic hydrocarbon derivatives of a sugar such as sucrose, sorbitol and lactose, a peptide such as a casein-derived peptide, an egg white-derived peptide, a soybean-derived peptide and glutathione, or a nucleic acid such as DNA, RNA, plasmid, siRNA and ODN.

Examples of the aliphatic hydrocarbon derivative of a water-soluble polymer include aliphatic hydrocarbon derivatives of polyethylene glycol, polyglycerin, polyethyleneimine, polyvinyl alcohol, polyacrylic acid, polyacrylamide, oligosaccharide, dextrin, water-soluble cellulose, dextran, chondroitin sulfate, polyglycerin, chitosan, polyvinylpyrrolidone, polyaspartic acid amide, poly-L-lysine, mannan, pullulan, oligoglycerol or the like or a derivative thereof, more preferably aliphatic hydrocarbon derivatives of a polyethylene glycol derivative or a polyglycerin derivative, and further preferably aliphatic hydrocarbon derivatives of a polyethylene glycol derivative.

In addition, examples of the lipid used in the bilayer lipid membrane include a synthetic lipid. Examples of the synthetic lipid include fluorinated phosphatidylcholine, fluorinated surfactant and dialkyl ammonium bromide, and these may be used alone, or may be used by combining with other lipid.

In addition, the bilayer lipid membrane contains preferably a lipid derivative, a fatty acid derivative or an aliphatic hydrocarbon derivative of a water-soluble substance, or the surfactant, more preferably a lipid derivative or a fatty acid derivative of the water-soluble polymer, further preferably the polyethylene glycolated phospholipid, and most preferably polyethylene glycol phosphatidyl ethanolamine.

Examples of the lipid derivative, the fatty acid derivative or the aliphatic hydrocarbon derivative of a water-soluble substance in the present invention include a lipid derivate or a fatty acid derivative of one or more substance(s) selected from sugar, peptide, nucleic acid and water-soluble polymer, and an aliphatic hydrocarbon derivative of one or more substance(s) selected from sugar, peptide, nucleic acid and water-soluble polymer, preferably a lipid derivative or a fatty acid derivative of a glycolipid or a water-soluble polymer, and more preferably a lipid derivative or a fatty acid derivative of a water-soluble polymer.

Examples of the aliphatic hydrocarbon derivative of a water-soluble substance include those in which a water-soluble substance is bound to, for example, an alcoholic residue of a long chain aliphatic alcohol, polyoxypropylenealkyl or glycerin fatty acid ester.

Examples of the aliphatic hydrocarbon derivative of a sugar, a peptide or a nucleic acid include aliphatic hydrocarbon derivatives of a sugar such as sucrose, sorbitol and lactose, a peptide such as a casein-derived peptide, an egg white-derived peptide, a soybean-derived peptide and glutathione, or a nucleic acid such as DNA, RNA, plasmid, siRNA and ODN.

Examples of the aliphatic hydrocarbon derivative of a water-soluble polymer include aliphatic hydrocarbon derivatives of polyethylene glycol, polyglycerin, polyethyleneimine, polyvinyl alcohol, polyacrylic acid, polyacrylamide, oligosaccharide, dextrin, water-soluble cellulose, dextran, chondroitin sulfate, polyglycerin, chitosan, polyvinylpyrrolidone, polyaspartic acid amide, poly-L-lysine, mannan, pullulan, oligoglycerol or a derivative thereof, more preferably aliphatic hydrocarbon derivatives of a polyethylene glycol derivative or a polyglycerin derivative, and further preferably aliphatic hydrocarbon derivatives of a polyethylene glycol derivative.

In the case where the lead particle is a fine particle comprising liposome B as a constituent component, a substance which comprises complex particles comprising the liposome B and the above-mentioned RNA as constituent components and a bilayer lipid membrane for coating the complex particles becomes liposome A, which is classified into liposome in a narrow sense based on its structure. Even if the lead particle is different from a fine particle comprising the liposome B as a constituent component, the lead particle is coated with a bilayer lipid membrane, therefore, the resulting substance is classified into liposome in a wide sense. In the present invention, it is more preferred that the constituent component of the lead particle is also liposome.

The complex particles comprising the lead particle and the RNA as constituent components in the present invention can be produced by adhering or encapsulating the RNA to or into the lead particle after or concurrently with the production of the lead particle. Further, liposome A can be produced by coating the complex particles with the bilayer lipid membrane after or concurrently with the production of the complex particles. The liposome A can be produced by or in accordance with a known production method described in, for example, Published Japanese translation of a PCT international application No. 2002-508765, Published Japanese translation of a PCT international application No. 2002-501511, "Biochimica et Biophysica Acta", Vol. 1510, pp. 152-166 (2001), and International Publication No. WO 02/28367, or can be produced by a production method including a step of dispersing the complex particles and coating layer components in a liquid which contains a polar organic solvent in which the coating layer components are soluble at a concentration at which the complex particles are not dissolved and the coating layer components are present in a dispersed state after the complex particles are produced by adhering or encapsulating the RNA to or into the lead particle, and a step of coating the complex particles with the coating layer components.

As a preferred production method of the liposome A in the composition of the present invention, the following production method including a step of producing complex particles comprising as constituent components a lead particle and the RNA (step 1) and a step of coating the complex particles with a bilayer lipid membrane (step 2 or step 3) can be exemplified.

### Step 1) Step of producing complex particles comprising as constituent components a lead particle and the RNA

Lead particles are dispersed in a solvent such as water, the RNA is dispersed or dissolved by mixing so as to be contained in the liquid in which the lead particles are dispersed, and the RNA is adhered to the lead particles. In the step 1, in order to suppress the aggregation of the lead particles, the lead particles are preferably lead particles comprising an aggregation-suppressing substance, and more preferably the lead particles contain as the aggregation-suppressing substance, the above-mentioned lipid derivative or fatty acid derivative of one or more substance(s) selected from sugars, peptides, nucleic acids and water-soluble polymers or the surfactant. Further, in the case where the lead particles have a positive electric charge, the RNA and an adhesion-competitive agent are allowed to coexist in the liquid in which the lead particles are dispersed, and the adhesion-competitive agent may be adhere to the lead particles as well as the RNA. Further, also in the case where the lead particles are lead particles comprising the aggregation-suppressing substance, in order to further suppress the aggregation of the lead particles, the adhesion-competitive agent may be used. In the combination of the lead particles and the RNA, it is preferred that a combination in which the complex particles are dispersible in the liquid containing a polar organic solvent is selected, and it is more preferred that the solubility of the complex particles in the polar organic solvent is lower than that of the constituent components of the bilayer lipid membrane to be used in the step 2 or 3. It is further more preferred that a combination in which the polar organic solvent can be contained in a liquid at such a concentration that the constituent components of the bilayer lipid membrane are dispersible and the complex particles are dispersible, is selected.

Examples of the adhesion-competitive agent include an anionic substance and the like, and the anionic substance includes a substance electrostatically adhered to the constituent components of the lead particles due to the electrostatic attraction by an electric charge, intramolecular polarization or the like in the molecule. The anionic substance as the adhesion-competitive agent is a substance exhibiting an anionic nature, however, even if it is an amphoteric substance having both cationic group and anionic group, the relative electronegativity varies depending on the pH, binding to other substance(s) or the like, therefore, the amphoteric substance can be classified into an anionic substance as the case may be.

Examples of the anionic substance include an anionic lipid, an anionic surfactant (the same definition as above), an anionic polymer and the like, a protein or a peptide, with which a complex can be formed at a pH equal to or greater than an isoelectric point, a nucleic acid and the like. Preferred examples thereof include one or more substance (s) selected from dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic, and the like. These anionic substances can be used alone, or can be used by combining two or more kinds.

Examples of the anionic lipid include phosphatidylserine, phosphatidylglycerol, phosphatidylinositol, phosphatidic acid and the like.

Examples of the anionic polymer include polyaspartic acid, a copolymer of styrene with maleic acid, a copolymer of isopropylacrylamide with acrylpyrrolidone, PEG-modified dendrimer modified polyeyhylene glycol, polylactic acid, polylactic acid polyglycolic acid, polyethylene glycolated polylactic acid, dextran sulfate, sodium dextran sulfate, chondroitin sulfate, sodium chondroitin sulfate, hyaluronic acid, chondroitin, dermatan sulfate, heparan sulfate, heparin, keratan sulfate, dextran fluorescein anionic and the like.

The protein or the peptide with which a complex can be formed at a pH equal to or greater than an isoelectric point is not particularly limited as long as it is a protein or a peptide with which a complex can be formed at a pH equal to or greater than the isoelectric point of the substance. Examples thereof include albumin, orosomucoid, globulin, fibrinogen, histone, protamine, ribonuclease, lysozyme and the like.

Examples of the nucleic acid as an anionic substance include DNA, RNA, plasmid, siRNA, ODN and the like. It may have any length and any sequence as long as it does not exhibit a physiological activity.

The adhesion-competitive agent is preferably electrostatically adhered to the constituent components of the lead particles, and is preferably a substance with a size which does not allow the crosslinking formation to aggregate the constituent components of the lead particles even if the substance is adhered to the constituent components of the lead particles, or a substance having in its molecule, a moiety which is adhered to the constituent components and a moiety which repels the adhesion and suppresses the aggregation of the lead particles.

More specifically, the step 1 can be carried out, for example, in a production method including a step of producing a liquid in which lead particles comprising an aggregation-suppressing substance are dispersed, and a step of dispersing or dissolving the RNA so as to be contained in the liquid in which the lead particles are dispersed (for example, a step of adding the RNA to the liquid in which the lead particles are dispersed and dispersing or dissolving the RNA therein, a step of adding a liquid in which the RNA is dispersed or dissolved to the liquid in which the lead particles are dispersed or the like). Here, specific examples of the complex particles obtained by the step of dispersing or dissolving the RNA so as to be contained in the liquid in which the lead particles are dispersed, include complex particles formed by adhering the RNA to fine particles comprising as a constituent component, liposome B comprising the cationic lipid, complex particles formed by adhering the RNA to fine particles comprising as a constituent component, a lipid assembly comprising the cationic lipid, and complex particles formed by adhering the RNA to fine particles comprising as a constituent component, a polymer comprising a cationic polymer such as poly-L-lysine. Further, the step of dispersing or dissolving the RNA so as to be contained in the liquid in which the lead particles are dispersed is preferably a step of further incorporating the adhesion-competitive agent in the liquid in which the RNA is dispersed or dissolved and adding the resulting liquid to the liquid in which the lead particles are dispersed. In this case, the complex particles are produced by adhering both of the RNA and the adhesion-competitive agent to the lead particles, and the production can be carried out by further suppressing aggregation of the lead particles during the production of the complex particles and aggregation of the complex particles after the production.

The ratio of the lead particles to the liquid in which the lead particles are dispersed is not particularly limited as long as the RNA can be adhered to the lead particles, however, it is preferably about 1 µg/mL to 1 g/mL, more preferably about 0.1 to 500 mg/mL.

### Step 2) Step of coating complex particles with bilayer lipid membrane (1)

Liposome A can be produced by, for example, a production method including a step of preparing a liquid (liquid A) containing a polar organic solvent in which the complex particles obtained in the step 1 are dispersed and the constituent components of the bilayer lipid membrane are dissolved, and a step of coating the complex particles with the bilayer lipid membrane by reducing the ratio of the polar organic solvent in the liquid A. In this case, the liposome A is obtained in the form of a dispersion (liquid B). The solvent in the liquid A is a solvent which contains a polar organic solvent at such a concentration that the constituent components of the bilayer lipid membrane are soluble and the complex particles are dispersible. In the liquid B in which the ratio of the polar organic solvent to the liquid A is reduced, the constituent components of the bilayer lipid membrane are dispersible and the complex particles are also dispersible. In the case where the solvent in the liquid A is a liquid mixture of a polar organic solvent and a solvent different from a polar organic solvent, for example, by adding a solvent comprising a solvent different from a polar organic solvent mixable with the polar organic solvent (liquid C), and/or selectively removing the polar organic solvent by distillation by evaporation, semipermeable membrane separation, fractional distillation or the like, the ratio of the polar organic solvent can be reduced. Here, the liquid C is a solvent comprising a solvent different from a polar organic solvent, and may also contain a polar organic solvent as long as the ratio of the polar organic solvent is lower than that of the polar organic solvent contained in the liquid A.

Examples of the solvent different from a polar organic solvent in the step 2 include water, liquid carbon dioxide, a liquid hydrocarbon, a halogenated carbon, a halogenated hydrocarbon and the like, and preferred examples thereof include water. Further, the liquid A and the liquid C may contain an ion, a buffer component or the like. These solvents can be used alone, or can be used by combining two or more kinds.

The combination of a polar organic solvent with a solvent different from a polar organic solvent is preferably a combination of solvents that are mixable with each other and can be selected by considering the solubility of the complex particles and the constituent components of the bilayer lipid membrane in the solvents in the liquid A and the liquid B, and the liquid C. On the other hand, the complex particles preferably have a low solubility in any of the solvents in the liquid A and the liquid B, and the liquid C, and also preferably have a low solubility in any of a polar organic solvent and a solvent different from a polar organic solvent. The constituent components of the bilayer lipid membrane preferably have a low solubility in the solvent in the liquid B and the liquid C, and preferably have a high solubility in the solvent in the liquid A, and preferably have a high solubility in a polar organic solvent and preferably have a low solubility in a solvent different from a polar organic solvent. Here, the complex particles having a low solubility means that the elution of each component contained in the complex particles such as the lead particles, RNA and adhesion-competitive agent in the solvent is low, and even if the respective solubilities of the components are high, it is sufficient that the elution of each component becomes low due to the binding or the like between the respective components. For example, even in the case where the solubility of any of the components contained in the lead particles in the solvent in the liquid A is high, if the lead particles have a positive electric charge, and an electrostatic bond is formed due to an electric charge, intramolecular polarization or the like in the RNA, and the solubility of the component(s) in the solvent in the liquid A becomes low, the elution of the components in the complex particles is suppressed, whereby the solubility of the complex particles in the solvent in the liquid A can be lowered. That is, if the lead particles have a positive electric charge, the elution of the components of the complex particles is suppressed in the production of the liposome A, and an effect of improving the productivity and yield is imparted.

The ratio of the polar organic solvent in the liquid A is not particularly limited as long as it is a ratio at which the constituent components of the bilayer lipid membrane are soluble and the complex particles are dispersible, and varies depending on the solvent, the complex particles to be used, the type of constituent components of the bilayer lipid membrane or the like. However, it is preferably 30 v/v% or more, more preferably 60 to 90 v/v%. Further, the ratio of the polar organic solvent in the liquid B is not particularly limited as long as the liquid B contains the polar organic solvent at a concentration lower than the liquid A and it is a ratio at which the constituent components of the bilayer lipid membrane are dispersible and the complex particles are also dispersible, however, it is preferably 50 v/v% or less.

The step of preparing the liquid A may be a step of preparing the liquid A by adding the polar organic solvent, the complex particles and the constituent components of the bilayer lipid membrane, or by adding the solvent different from the polar organic solvent. The polar organic solvent, the complex particles, the constituent components of the bilayer lipid membrane and the solvent different from the polar organic solvent can be added in any order as long as the complex particles are not dissolved. Preferably, a step of preparing the liquid A by preparing a liquid (liquid D) containing a polar organic solvent in which the complex particles are dispersed, preparing a liquid (liquid E) in which the constituent components of the bilayer lipid membrane are dissolved in a solvent containing a polar organic solvent that is the same as or different from the polar organic solvent in the liquid D and mixing the liquid D and the liquid E can be exemplified. When the liquid A is prepared by mixing the liquid D and the liquid E, it is preferred to mix them gradually.

### Step 3) Step of coating complex particles with bilayer lipid membrane (2)

Liposome A can be produced by a production method including a step of dispersing the complex particles obtained in the step 1 and the constituent components of the bilayer lipid membrane in a liquid (liquid F) which contains a polar organic solvent in which the constituent components of the bilayer lipid membrane are soluble at a concentration at which the complex particle are not dissolved and the constituent components of the bilayer lipid membrane are present in a dispersed state. In this case, the liposome A can be obtained in a state of a dispersion. The solvent in the liquid F is a solvent which contains a polar organic solvent in which the constituent component of the bilayer lipid membrane is soluble, and the polar organic solvent is contained in the liquid F at such a specific concentration that both of the constituent component of the bilayer lipid membrane and the complex particles are dispersible.

As a method of preparing the liquid F, any embodiment can be employed. For example, the liquid F may be prepared by preparing a dispersion of complex particles and a solution or a dispersion of the constituent components of the bilayer lipid membrane and mixing both liquids, or the liquid F may be prepared by preparing either one of the dispersions of the complex particles and the constituent components of the bilayer lipid membrane, and adding and dispersing the other remaining complex particles or constituent components of the bilayer lipid membrane in the form of a solid in the resulting dispersion. In the case where a dispersion of the complex particles and a solution or a dispersion of the constituent components of the bilayer lipid membrane are mixed, a dispersion medium of the complex particles may contain a polar organic solvent in advance, further, the constituent components of the bilayer lipid membrane may be dissolved or dispersed therein, and a solvent or a dispersion medium of the constituent components of the bilayer lipid membrane may be a liquid containing a polar organic solvent or a liquid composed only of a polar organic solvent. On the other hand, in the case where the dispersions of either one of the complex particles and the constituent components of the bilayer lipid membrane is prepared, and the other remaining complex particles or constituent components of the bilayer lipid membrane in the form of a solid are added to the resulting dispersion, the resulting dispersion is a liquid containing a polar organic solvent. Incidentally, in the case where the complex particles are not dissolved and the constituent components of the bilayer lipid membrane are dispersed after the liquid F is prepared, a polar organic solvent may be added in a concentration range of the polar organic solvent in which the complex particles are not dissolved and the constituent components of the bilayer lipid membrane are dispersed, or the organic solvent may be removed or the concentration thereof may be reduced. On the other hand, in the case where the complex particles are not dissolved and the constituent components of the bilayer lipid membrane are dissolved after the liquid F is prepared, the polar organic solvent may be removed or the concentration thereof may be reduced in a concentration range of the polar organic solvent in which the complex particles are not dissolved and the constituent components of the bilayer lipid membrane are dispersed. Alternatively, the complex particles and the constituent components of the bilayer lipid membrane are mixed in a solvent different from a polar organic solvent in advance, and a polar organic solvent may be added thereto in a concentration range of the polar organic solvent in which the complex particles are not dissolved and the constituent components of the bilayer lipid membrane are dispersed. In this case, the complex particles and the constituent components of the bilayer lipid membrane are separately dispersed in solvents different from a polar organic solvent, and both dispersions are mixed, and then, a polar organic solvent may be added thereto, or either one of the complex particles and the constituent components of the bilayer lipid membrane are dispersed in a solvent different from a polar organic solvent, and the other remaining complex particles or constituent components of the bilayer lipid membrane in the form of a solid are added to the resulting dispersion, and then a polar organic solvent may be added thereto. Further, it is preferred to include a step of letting a liquid, in which the complex particles and the constituent components of the bilayer lipid membrane are dispersed and a polar organic solvent is contained, stand or mixing the liquid for a time sufficient to coat the complex particles with the bilayer lipid membrane. The time for letting the liquid stand or mixing the liquid after the complex particles and the constituent components of the bilayer lipid membrane are dispersed in the liquid comprising the polar organic solvent is not limited as long as it is not completed immediately after the complex particles and the constituent components of the bilayer lipid membrane are dispersed in the liquid comprising the polar organic solvent, however, it can arbitrarily be set depending on the constituent components of the bilayer lipid membrane or the type of the liquid comprising the polar organic solvent, and it is preferably be set to a time which keeps the yield of the obtained liposome A constant, for example, about 3 seconds to 30 minutes.

Examples of the solvent different from a polar organic solvent in the liquid F include those illustrated in the solvent different from a polar organic solvent in the step 2, and preferred examples thereof include water.

The ratio of the polar organic solvent in the liquid F is not particularly limited as long as only the requirement that both of the complex particles and the constituent components of the bilayer lipid membrane are dispersed is met, and varies depending on the solvent or the complex particles to be used, the type of the constituent components of the bilayer lipid membrane or the like. However, it is preferably about 1 to 80 vol%, more preferably about 10 to 60 vol%, more preferably about 20 to 50 vol%, and the most preferably about 30 to 40 vol%.

In the present invention, the description of the constituent components of the bilayer lipid membrane being soluble in a polar organic solvent include a case in which the constituent components of the bilayer lipid membrane have a property of being dissolved in a polar organic solvent, a case in which the constituent components of the bilayer lipid membrane have a property of being dissolved in a polar organic solvent with the help of a solubilizer or the like, a case in which the constituent components of the bilayer lipid membrane have a property capable of being emulsified or formed into an emulsion by forming aggregates, micelles or the like in a polar organic solvent and the like. Further, the description of the constituent components of the bilayer lipid membrane being dispersible includes a state in which the whole of the constituent components of the bilayer lipid membrane form aggregates, micelles or the like and are emulsified or formed into an emulsion, a state in which a part of the constituent components of the bilayer lipid membrane form aggregates, micelles or the like and are emulsified or formed into an emulsion, and the rest of the components are dissolved, a state in which a part of the constituent components of the bilayer lipid membrane form aggregates, micelles or the like and are emulsified or formed into an emulsion, and the rest of the components are precipitated and the like. Incidentally, the description of the constituent components of the bilayer lipid membrane being dissolved does not include a state in which the whole of the constituent components of the bilayer lipid membrane form aggregates, micelles or the like and are emulsified or formed into an emulsion.

In the present invention, the description of the complex particles being dispersed means a state in which the complex particles are suspended or emulsified or formed into an emulsion, and includes a state in which a part of the complex particles are suspended or emulsified or formed into an emulsion, and the rest of the particles are dissolved, a state in which a part of the complex particles are emulsified or formed into an emulsion, and the rest of the particles are precipitated and the like. The description of the complex particles being not dissolved is the same as the above-mentioned definition of the complex particles being dispersed.

The concentration of the complex particles in the liquid containing a polar organic solvent to be used in the method of producing liposome A according to the present invention is not particularly limited as long as it allows the complex particles to be coated with the bilayer lipid membrane, however, it is preferably about 1 µg/mL to 1 g/mL, more preferably about 0.1 to 500 mg/mL. Further, the concentration of the components of the bilayer lipid membrane to be used is not particularly limited as long as it allows the complex particles to be coated, however, it is preferably about 1 µg/mL to 1 g/mL, more preferably about 0.1 to 400 mg/mL.

The ratio of the bilayer lipid membrane to the liposome A of the present invention is preferably about 1:0.1 to 1:1000, more preferably about 1:1 to 1:10 in ratio by weight.

Further, as for the size of the liposome A of the present invention, an average particles diameter is preferably about 300 nm or less, more preferably about 200 nm or less. Specifically, for example, an injectable size is preferred.

Further, the liposome A obtained above can be modified with a substance such as a protein including an antibody and the like, a saccharide, a glycolipid, an amino acid, a nucleic acid or any of various low-molecular compounds and polymers, and such coated complex particles obtained by modification is included in the liposome A. For example, in order to apply to targeting, it is possible that the liposome A obtained above is further subjected to a surface modification of the bilayer lipid membrane using a protein such as an antibody, a peptide, a fatty acid or the like (see "Stealth Liposome", edited by D. D. Lasic and F. Martin, CRC Press Inc., USA, pp. 93-102, (1995)). Further, surface improvement can also be optionally carried out to the liposome A using, for example, a lipid derivative, a fatty acid derivative or an aliphatic hydrocarbon derivative, of a water-soluble substance. The lipid derivative, the fatty acid derivative and the aliphatic hydrocarbon derivative of a water-soluble substance to be used in the surface modification are the same definition as the lipid derivative, the fatty acid derivative and the aliphatic hydrocarbon derivative of a water-soluble substance as the constituting components of the bilayer lipid membrane.

By administering the composition of the present invention to a mammal including a human, the RNA can be delivered to an eyeball which is an expression site of a target gene, RNA suppressing expression of, for example, VEGF gene, VEGFR gene, KLF5 gene and a gene whose transcription is activated by KLF5 can be introduced into a mammalian cell in vivo, thereby, expression of KLF5 gene and a gene whose transcription is activated by KLF5 and the like can be suppressed. By the composition of the present invention, for example, the expression of KLF5 gene and a gene whose transcription is activated by KLF5 is suppressed, and the proliferation of a vascular smooth muscle and angiogenesis can be suppressed, therefore, the composition of the present invention can be used as a medicament for treating or preventing of a disease in an eyeball such as diabetic retinopathy, retinopathy of prematurity or age related macular degeneration associated with proliferation of a vascular smooth muscle and angiogenesis. In addition, a composition in which the RNA in the composition of the present invention is replaced with a peptide or a protein such as a coenzyme and an antibody, nucleic acid such as oligonucleotide and plasmid can similarly deliver to an eyeball, and can be used as a medicament for treating or preventing of a disease in an eyeball.

That is, the present invention also provides a method of treating a disease in an eyeball comprising administering the composition of the present invention explained above to a mammal. A subject to be administered is preferably a human, more preferably a human suffering from a disease in an eyeball.

In addition, the composition of the present invention, and a composition in which the RNA in the composition of the present invention is replaced with a peptide or a protein such as a coenzyme and an antibody, a nucleic acid such as oligonucleotide and plasmid can be used as a tool for acquiring POC (Proof of concept) in an in vivo screening system regarding a medicament for treating or preventing of a disease in an eyeball.

The composition of the present invention, and a composition in which the RNA in the composition of the present invention is replaced with a peptide or a protein such as a coenzyme and an antibody, a nucleic acid such as oligonucleotide and plasmid can be also used as a preparation for the purpose of, for example, for stabilization of the RNA, the peptide, the protein or the nucleic acid in a living body component such as blood components (e.g. blood, gastrointestinal tract etc.), for reduction of the side effect, for increase in the accumulation property of a drug in an eyeball or the like.

In the case where the composition of the present invention, and a composition in which the RNA in the composition of the present invention is replaced with a peptide or a protein such as a coenzyme and an antibody, a nucleic acid such as oligonucleotide and plasmid is used as a medicament for treating or preventing of a disease in an eyeball of a medicament, according to an administration route, it is desirable to use an administration route that is most effective for treatment is used. Examples of the administration route include parenteral or oral administration routes such as intraoral administration, tracheobronchial administration, intrarectal administration, subcutaneous administration, intramuscular administration, and intravenous administration, preferably intravenous administration and intramuscular administration, more preferably intravenous administration.

The dose is different depending on a symptom and an age of a subject to be administered, and administration routes, for example, one day dose in terms of RNA may be administered so as to be about 0.1µg to 1000mg.

As a preparation suitable for intravenous administration or intramuscular administration, for example, an injection can be exemplified, and it is also possible to use the dispersion of the liposome A prepared by the above-mentioned method as it is in the form of, for example, an injection or the like. However, it can also be used after removing the solvent from the dispersion by, for example, filtration, centrifugation or the like, or after lyophilizing the dispersion or the dispersion supplemented with an excipient such as mannitol, lactose, trehalose, maltose or glycine.

In the case of an injection, it is preferred that an injection is prepared by mixing, for example, water, an acid, an alkali, any of various buffers, a physiological saline solution, an amino acid infusion or the like with the dispersion of the liposome A or the liposome A obtained by removing the solvent or lyophilization. Further, it is possible to prepare an injection by adding an antioxidant such as citric acid, ascorbic acid, cysteine or EDTA, an isotonic agent such as glycerol, glucose or sodium chloride or the like. Further, it can also be cryopreserved by adding a cryopreservation agent such as glycerol.

Examples of a medicament for treating of a disease in an eyeball of the present invention include a medicament for treating of a disease in an eyeball comprising, among the liposome A, for example, a liposome composing a complex particle comprising a lead particle and the RNA as constituent components; and a bilayer lipid membrane for coating the complex particle; wherein the constituent components of the bilayer lipid membrane are soluble in a polar organic solvent, and the constituent components of the bilayer lipid membrane and the complex particle are dispersible in a liquid comprising the polar organic solvent at a specific concentration, or a liposome composing a complex particle comprising a lead particle and the RNA as constituent components, the lead particle comprising a cationic lipid; and a bilayer lipid membrane for coating the complex particle; wherein the constituent components of the bilayer lipid membrane are soluble in a polar organic solvent, and the constituent components of the bilayer lipid membrane and the complex particle are dispersible in a liquid containing the polar organic solvent at a specific concentration.

In addition, the present invention also provides use of the composition of the present invention explained above for production of a medicament for treating of a disease in an eyeball.

Then, the present invention will be specifically explained by way of Examples and Test Examples. However, the present invention is not limited by Examples and Test Examples. RNA used in Example 1 was prepared by obtaining RNA in which each 5' terminus of a single-stranded RNA of KLF5siRNA No.4 among KLF5siRNA in Table 1 is modified with carboxyfluorescein (FAM) and UU added to each 3' terminus is replaced with dTdT (FAM-AAGCUCACCUGAGGACUCAdTdT (SEQ ID No.: 23), FAM-UGAGUCCUCAGGUGAGCUUdTdT (SEQ ID No.: 24)) from Hokkaido System Science, and annealing this (hereinafter, referred to as FAM-labeled siRNA). In addition, RNA used in Example 2 is RNA comprising a sequence of 19 contiguous nucleotides of a rat KLF gene mRNA and a sequence complementary to the sequence (5'-GCAUCAACAUGAACGUCUU-3' (SEQ ID No.: 25), 5'-AAGACGUUCAUGUUGAUGCUG-3' (SEQ ID NO.: 26)) (hereinafter, referred to as KLF5siRNA-2), and RNA used in Example 3 is RNA comprising a sequence of 19 contiguous nucleotides of VEGFR1 gene mRNA and a sequence complementary to the sequence (5'-CUGAGUUUAAAAGGCACCCdTdT-3' (SEQ ID No.: 27), 5'-GGGUGCCUUUUAAACUCAGdTdT-3' (SEQ ID No.: 28)) (hereinafter, referred to as VEGFR1siRNA), each of which was prepared by obtaining from Hokkaido System Science, and annealing this.

### Example 1

DOTAP (manufactured by Avanti Polar Lipids Inc. ) /PEG-DSPE (manufactured by NOF Co. , Ltd. , hereinafter the same) /distilled water were mixed at 30 mg/12 mg/mL, and shaken and stirred with a vortex mixer. The resulting dispersion was passed through a 0.4 µm polycarbonate membrane filter (manufactured by Whatmann) for four times, a 0.1 µm polycarbonate membrane filter (manufactured by Whatmann) for ten times and, further, a 0.05 µm polycarbonate membrane filter (manufactured by Whatmann) for 24 times to prepare a lead particle. To 0.18 mL of a dispersion of the resulting lead particle was added 0.09 mL of an 8 mg/mL aqueous solution of FAM-labeled siRNA, and 0.36 mL of ethanol was added to prepare complex particles. To a dispersion of the resulting complex particles was added 0.09mL of a solution in which EPC (manufactured by NOF Co., Ltd.)/PEG-DSPE which was a constituent component of a bilayer lipid membrane were dissolved in ethanol at 120 mg/25 mg/mL, then, 8.3 mL of distilled water was gradually added, and a concentration of ethanol was adjusted to 5 vol% to prepare a liposome. A dispersion of the resulting liposome was subjected to ultracentrifugation (1 hour, 110,000×g, 25°C), the supernatant was removed, and a physiological saline solution was added to re-disperse to obtain liposome dispersion. PEG-DSPE at 50 parts by weight based on 120 parts by weight of EPC was dissolved in a small amount (about 1/25 volume of liposome dispersion) of ethanol (PEG-DSPE ethanol solution). The liposome dispersion and the PEG-DSPE ethanol solution were heated at 70°C for 2 minutes, respectively. Then, the liposome dispersion was added to the PEG-DSPE ethanol solution, and this was mixed, heated at 70°C for 2 minutes, and cooled with water to obtain a preparation.

### Test Example 1

For example, using a disease model generating choroidal neovascularization such as age related macular degeneration (see Ryan SJ.Subretinal neovascularization after argon laser photocoagulation. Albrecht Von Graefes Arch Klin Exp Ophthalmol.1980;215(1):29-42), it was confirmed that the preparation obtained in Example 1 reached an eyeball by the following method.

Ketamine hydrochloride was intraperitoneally administered to a C57 BL/6j (10 to 12 week old) mouse, and general anesthesia was performed. Three places of mouse right fundus oculi were irradiated with semiconductor laser under the irradiation condition of a power of 200 mW, a coagulation time of 20 msec, and a coagulation diameter of 200 µm. It was confirmed that, at irradiation, bubbles appeared in fundus oculi, a Bruch's membrane was lost, and a retina was clouded. Five days after laser irradiation, 100 µL (corresponding to 150 µg of FAM-labeled siRNA) of the preparation obtained in Example 1 was administered through superficial dorsal vein of penis of a mouse, and fundus fluorescent angiography (ophthalmoscope TRC-50IX, TOPCON, Tokyo, Japan)(see Y.Yanagi, Y.Tamaki, R.Obata, K.Muranaka, N.Homma, H.Matsuoka, and H.Mano, Subconjunctival administration of bucillamine suppresses choroidal neovascularization in rat, Invest Ophthalmol Vis Sci 43(2002) 3495-3499) was performed for 1 hour, 2 hours, 4 hours, 8 hours, 24 hours (1 day), 48 hours (2 days), 72 hours(3 days), 96 hours (4 days), 120 hours (5 days), 144 hours (6 days) and 168 hours (7 days) after administration of the preparation. The resulting fundus fluorescent angiogram was converted into 8 bit, and an intensity of each image of a lesion was summed to calculate the accumulation of a FAM-labeled siRNA in a retina lesion site.

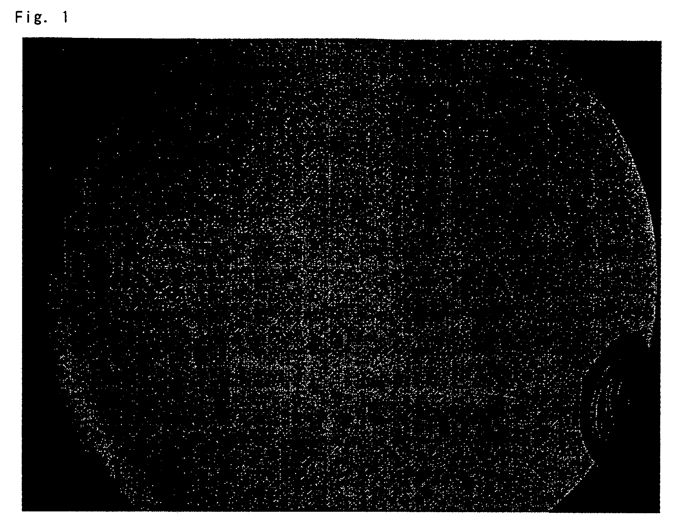

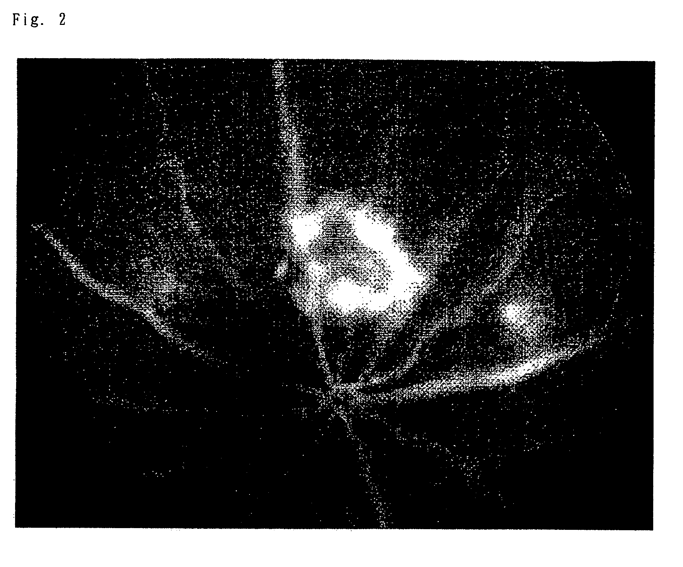

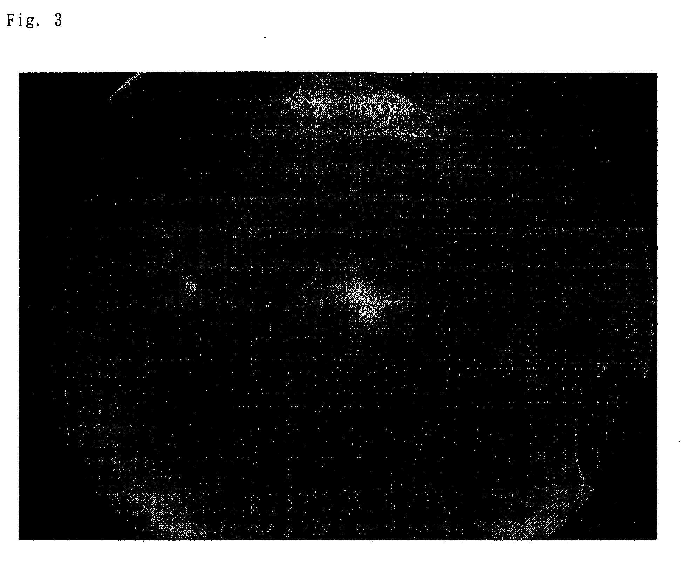

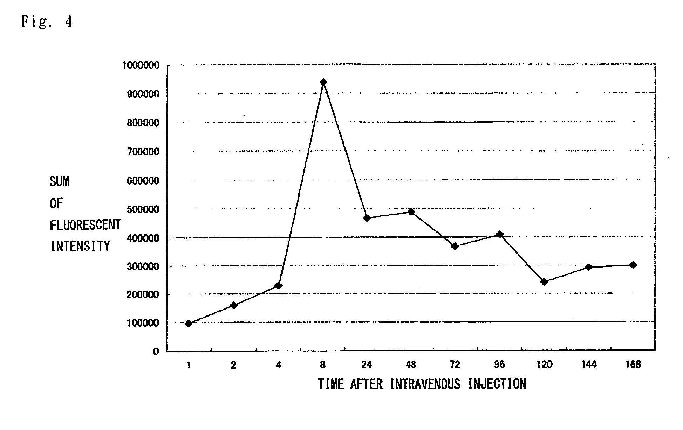

Figs. 1 to 3 show a fundus fluorescent angiogram after 2, 8 and 168 hours following administration of the preparation obtained in Example 1. In addition, Fig.4 shows a change in a time of accumulation of a FAM-labeled siRNA in a retina lesion site. In Figs.1 to 3, a normal blood vessel running radically from a center can be observed and further, three places of a disorder site due to laser damage are circularly imaged in a gap between normal blood vessels. FAM-labeled siRNA is imaged in a disorder site at a maximum intensity after 8 hours following administration, further, it can be observed that the siRNA is distributed in a disorder site also after 168 hours, and it has been made clear that the preparation obtained in Example 1 has reached an eyeball.

### Test Example 2

As in Test Example 1, mouse fundus oculi were irradiated with laser and, after five days, 100 µL of the preparation obtained in Example 1 (corresponding to 150 µg of FAM-labeled siRNA) was administered through superficial dorsal vein of penis of a mouse. A mouse was subjected to thoracotomy under general anesthesia after 2 hours, 4 hours and 7 days following administration, and right auricle was excised to remove blood. A 10% by mass solution (40 µL) of fluorescein isothiocyanate (FITC)-labeled lectin (Concanavalin A:VECT OR Laboratories Inc.(CA.USA) FL-1001) was diluted into 5 mL of PBS, filtered with a 40 µm filter, and injected and perfused into a left ventricle. Further, this was washed and perfused with 10 mL of PBS. An eyeball was isolated after 2, 4 and 168 hours following administration of the preparation obtained in Example 1, respectively, a step-serial frozen section having a thickness of 10 µm was made, and fluorescence derived from FITC and fluorescence derived from FAM were observed on the same field with a fluorescence microscope, respectively.

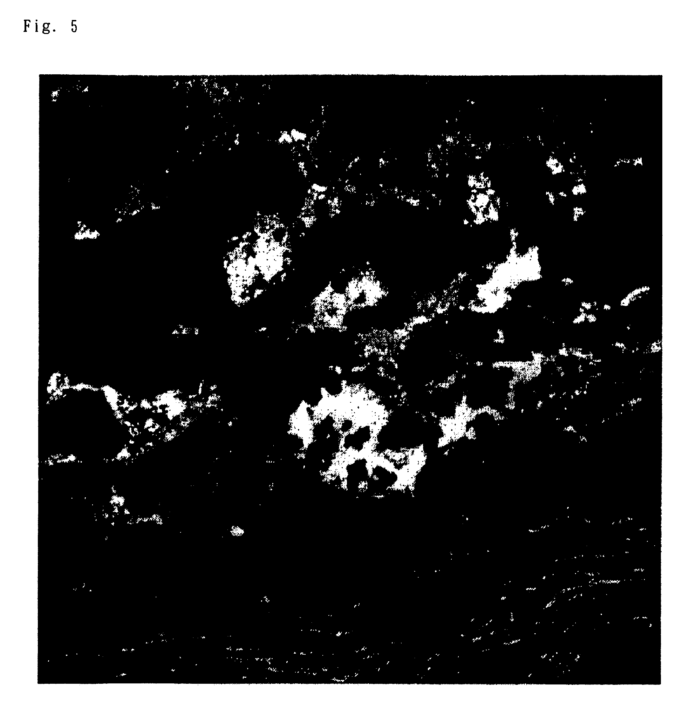

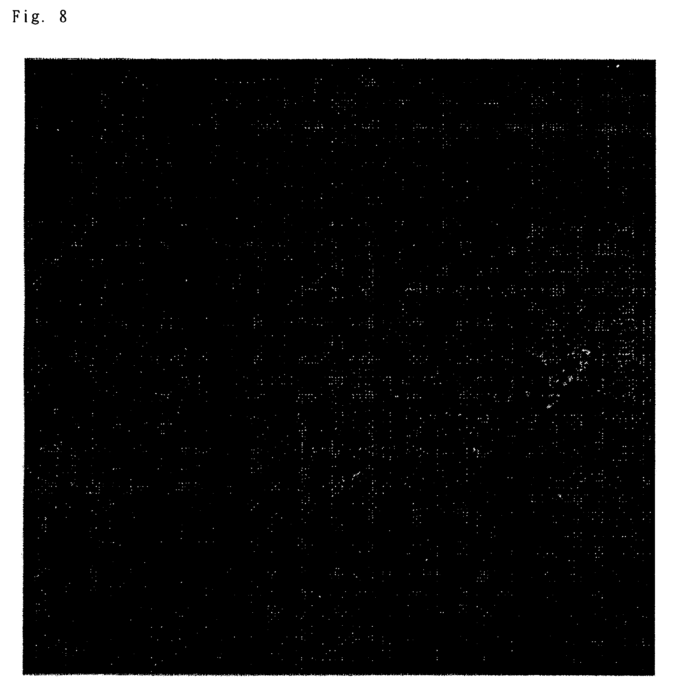

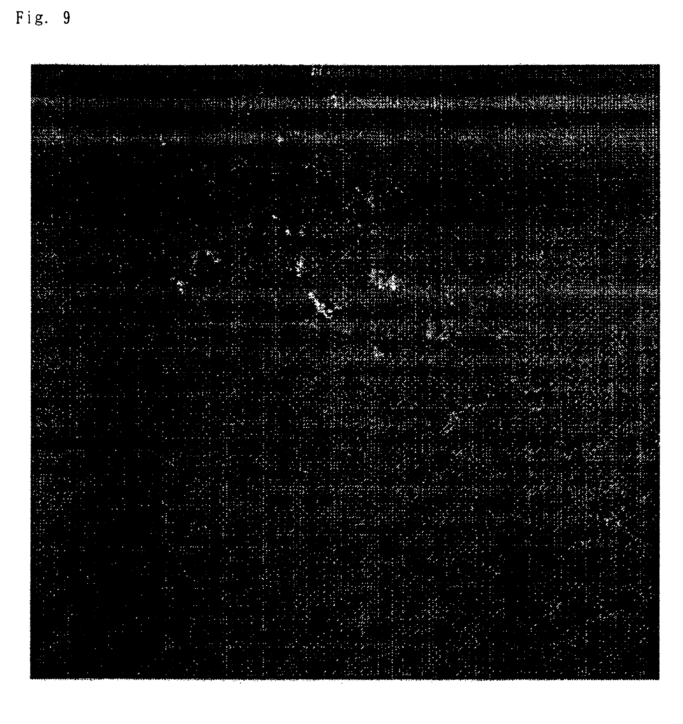

Figs. 5 to 7 show a fluorescent photograph derived from a retinal FITC after 2, 4 and 168 hours following administration of the preparation obtained in Example 1, respectively. Figs. 8 to 10 show a fluorescent photograph derived from a retinal FAM after 2, 4 and 168 hours following administration of the preparation obtained in Example 1, respectively.

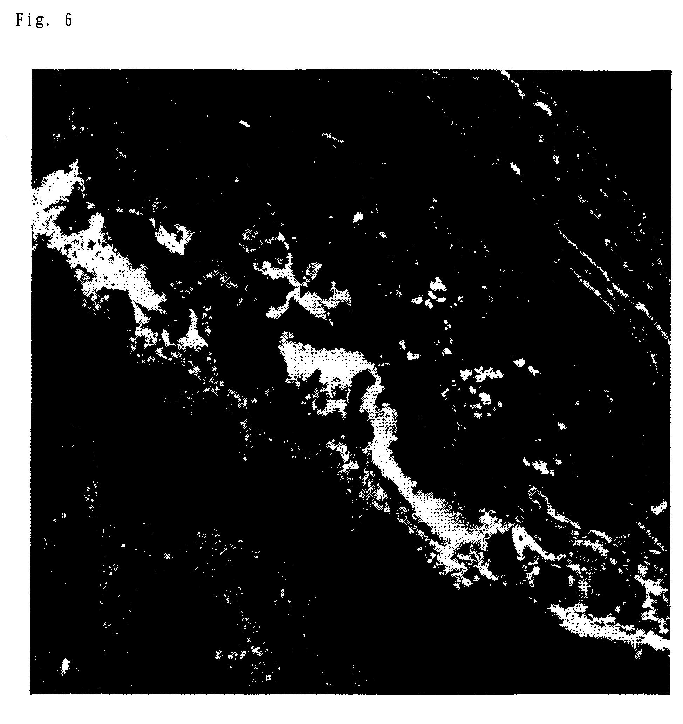

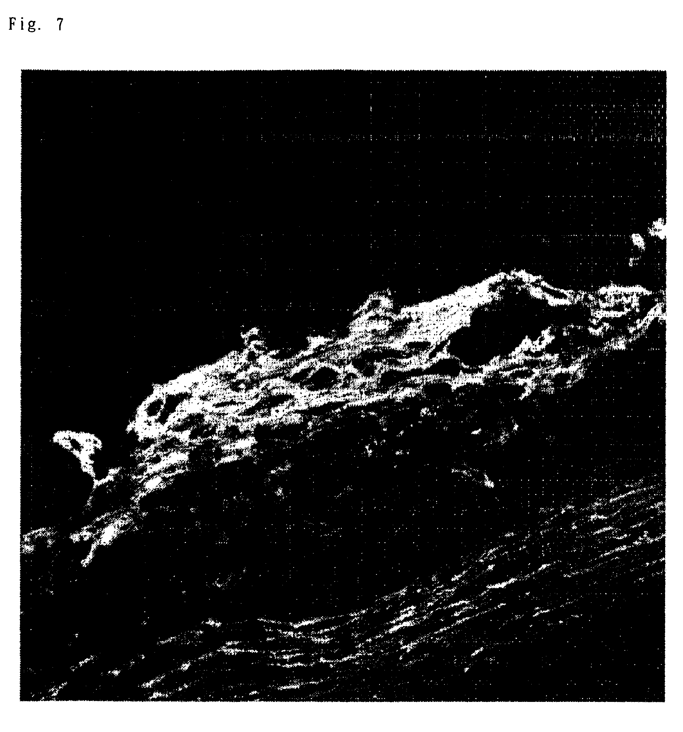

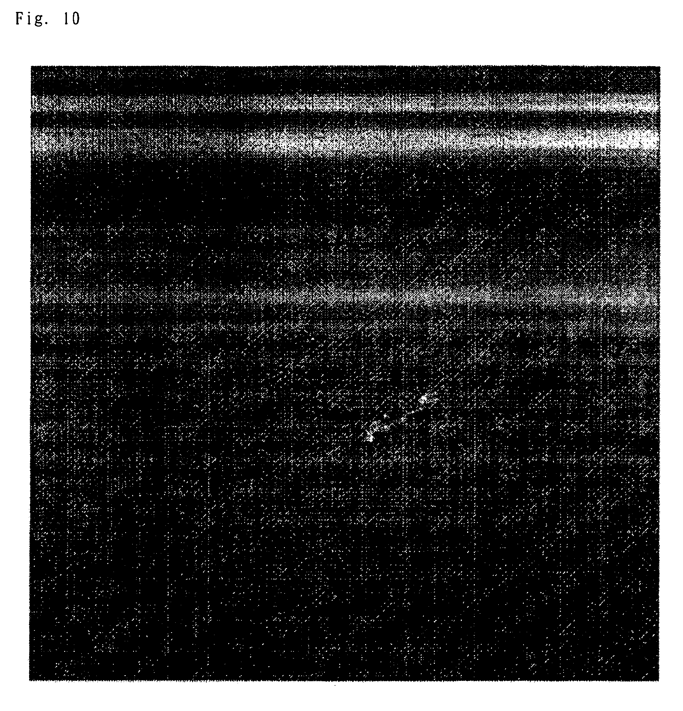

In Figs. 5 and 7, a retina is situated at upper position, and a choroidea and a sclera are situated at a lower position. In Fig.6, a retina is situated at a left lower position, and choroidea and a sclera are situated at a right upper position. A potion at which green fluorescence is strongly perceived shows the accumulation of FITC. Diffusely extending fluorescence derived from FITC is recognized in a choroidea and it is seen that a new born blood vessel is generated in a choroidea (see Antonia M.Joussen, Toshinori Murata, Akitaka Tsujikawa, Bernd Kirchho, Sven-Erik Bursell and Anthony P.Ada mis Leukocyte-Mediated Endothelial Cell Injury and Death in the Diabetic Retina American Journal of Pathology.2001;158:147-152). In Figs. 8, 9 and 10, an image is taken on the same specimen and the same field as those of Figs. 5, 6 and 7, respectively. In Figs. 8, 9 and 10, all of portions exhibiting fluorescence derived from FAM are distributed in a range of a fluorescence site derived from FITC indicating a new born blood vessel in Figs. 5, 6 and 7. In addition, from Figs. 7 and 10, it is seen that a FAM-labeled siRNA remains in a new born blood vessel site until 7 days following administration.

### Example 2

According to the same manner as that of Example 1 except that the FAM-labeled siRNA was replaced with KLF5siRNA-2, a preparation was obtained.

### Test Example 3

Using the same disease model as that of Test Example 1, it has been confirmed that the preparation obtained in Example 2 suppresses choroidal neovascularization by the following method.

Ketamine hydrochloride was intraperitoneally administered to a C57 BL/6j (10 to 12 week age) mouse, and general anesthesia was performed. Three places of mouse both fundus oculi were irradiated with semiconductor laser under the irradiation condition of a power of 200 mW, a coagulation of time of 20 msec, and a coagulation diameter of 200 µm. It was confirmed that, at irradiation, bubbles appeared in fundus oculi, a Bruch's membrane was lost, and a retina was clouded. At laser irradiation, after 24 hours (1 day) and after 48 hours (2 days), the preparation obtained in Example 2 was administered at 100 µL (corresponding to 100 µg of KLF5siRNA-2) per mouse weight 20 g every time through a mouse tail vein. After 72 hours (3 days) following initial administration, a solution in which 0.7% fluorescein was dissolved in a physiological saline solution was intraperitoneally administered at 20 µL (corresponding to 140 µg of fluorescein) per mouse weight 20 g every time, and fundus fluorescent angiography was performed. The resulting fundus fluorescent angiogram was converted into 8 bit, and a luminance intensity of each image of a lesion was summed to quantitate choroidia neovascularization. In addition, as a control, in place of the preparation obtained in Example 2, an aqueous physiological saline solution of KLF5 siRNA-2 was administered at 100 µL (corresponding to 100 µg of KLF5siRNA-2) per mouse weight 20 g every time, and the similar test was performed (control 1).

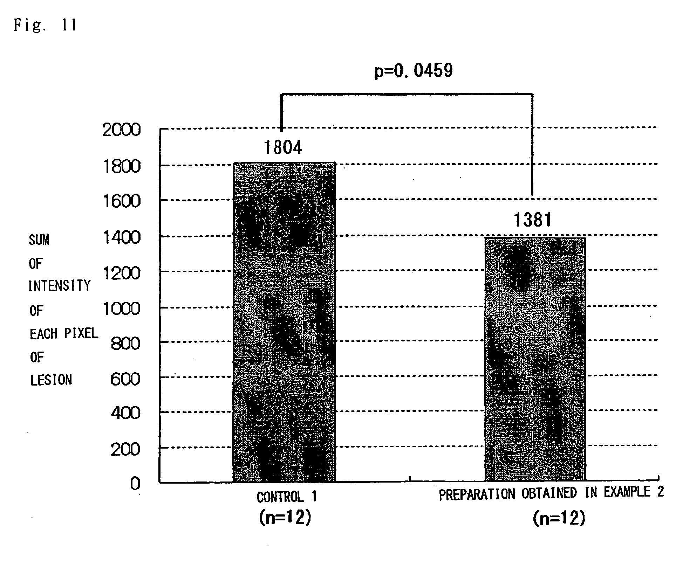

Fig.11 shows a bar graph showing choroidal neovascularization after 72 hours following initial administration. It has been made clear that administration of the preparation obtained in Example 2 significantly suppresses choroidal neovascularization as compared with a control 1, and is useful for a medicament for treating or preventing of a disease in an eyeball. The suppression rate relative to the control 1 was 23%, p=0.0459.

### Example 3

According to the same manner as that of Example 1 except that the FAM-labeled siRNA was replaced with VEGFR1siRNA, the preparation was obtained.

### Test Example 4

The preparation obtained in Example 3 was administered at 100 µL (corresponding to 100 µg of VEGFR1siRNA) per mouse weight 20 g every time, a physiological saline solution as a control was administered at 100 µL (control 2) per mouse weight 20 g every time, and an aqueous solution of VEGFR1siRNA in a physiological saline solution was administered at 100 µL (corresponding to 100 µg of VEGFR1siRNA) (control 3) per mouse weight 20 g every time, and the test was similarly performed.

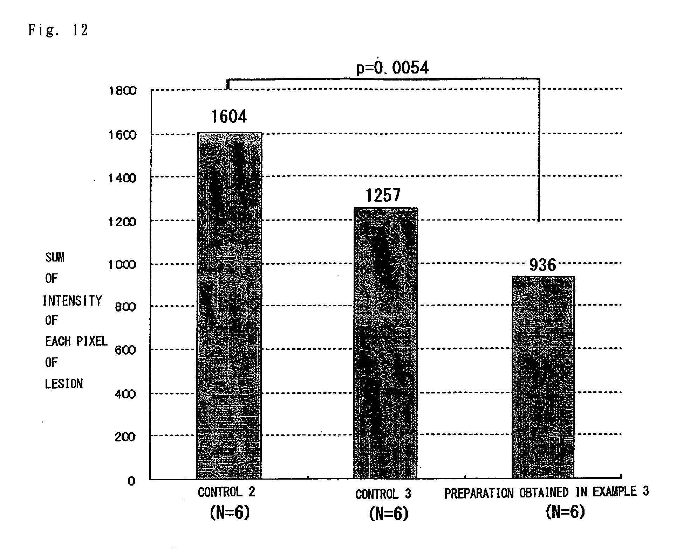

Fig.12 shows a bar graph showing suppression of choroidal neovascularization after 72 hours following initial administration. The preparation obtained in Example 3 significantly suppresses choroidal neovascularization as compared with controls 2 and 3, and it has been made clear that it is useful for a medicament for treating or preventing of a disease in an eyeball. The suppression rate relative to the control 2 was 42%, p=0.0054.

### INDUSTRIAL APPLICABILITY

The composition of the present invention is useful for a medicament for preventing or treating of a disease in an eyeball such as diabetic retinopathy, retinopathy of prematurity or age related macular degeneration.

## Claims

1. A composition comprising an RNA-encapsulated liposome, the RNA comprising a sequence being of 15 to 30 contiguous nucleotides of mRNA of a target gene associated with a disease in an eyeball, and a sequence complementary to the sequence,
wherein the liposome is capable of reaching an eyeball.

2. The composition according to claim 1, wherein the liposome is a liposome having a size that allows intravenous administration thereof.

3. The composition according to claim 1 or 2, wherein the RNA is RNA having an action of suppressing the expression of the target gene utilizing RNA interference (RNAi).

4. The composition according to any one of claims 1 to 3, wherein the target gene associated with a disease in an eyeball is a gene involved in angiogenesis in an eye disease.

5. The composition according to any one of claims 1 to 3, wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.

6. The composition according to any one of claims 1 to 3, wherein the mRNA of a target gene associated with a disease in an eyeball is KLF5 mRNA.

7. The composition according to any one of claims 1 to 6, wherein the mRNA is either human or mouse mRNA.

8. The composition according to any one of claims 1 to 7, wherein the RNA-encapsulated liposome is a liposome composing a complex particle comprising a lead particle and the RNA as constituent components; and a bilayer lipid membrane for coating the complex particle; and
wherein the constituent components of the bilayer lipid membrane are soluble in a polar organic solvent, and the constituent components of the bilayer lipid membrane and the complex particle are dispersible in a liquid containing the polar organic solvent at a specific concentration.

9. The composition according to claim 8, wherein the polar organic solvent is an alcohol.

10. The composition according to claim 8, wherein the polar organic solvent is ethanol.

11. The composition according to any one of claims 8 to 10, wherein the lead particle is a lead particle comprising a cationic lipid, and
wherein the bilayer lipid membrane is a bilayer lipid membrane comprising a neutral lipid, and a lipid derivative, a fatty acid derivative or an aliphatic hydrocarbon derivative of a water-soluble substance as constituent components.

12. The composition according to any one of claims 1 to 7, wherein the RNA-encapsulated liposome is a liposome composing a complex particle comprising a lead particle and the RNA as constituent components, the lead particle comprising a cationic lipid; and a bilayer lipid membrane for coating the complex particle; and
wherein the bilayer lipid membrane is a bilayer lipid membrane comprising a neutral lipid, and a lipid derivative, a fatty acid derivative or an aliphatic hydrocarbon derivative of a water-soluble substance as constituent components.

13. The composition according to claim 11 or 12, wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.

14. The composition according to any one of claims 11 to 13, wherein the lipid derivative, the fatty acid derivative or the aliphatic hydrocarbon derivative of a water-soluble substance is polyethylene glycol phosphatidyl ethanolamine.

15. The composition according to any one of claims 11 to 14, wherein the neutral lipid is egg yolk phosphatidylcholine.

16. A medicament for treating of a disease in an eyeball comprising an RNA-encapsulated liposome, the RNA-encapsulated liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
wherein the constituent components of the bilayer lipid membrane are soluble in a polar organic solvent, and the constituent components of the bilayer lipid membrane and the complex particle are dispersible in a liquid comprising the polar organic solvent at a specific concentration.

17. The medicament for treating of a disease in an eyeball according to claim 16, wherein the polar organic solvent is an alcohol.

18. The medicament for treating of a disease in an eyeball according to claim 16, wherein the polar organic solvent is ethanol.

19. The medicament for treating of a disease in an eyeball according to any one of claims 16 to 18, wherein the lead particle is a lead particle comprising a cationic lipid, and
wherein the bilayer lipid membrane is a bilayer lipid membrane comprising a neutral lipid, and a lipid derivative, a fatty acid derivative or an aliphatic hydrocarbon derivative of a water-soluble substance as constituent components.

20. A medicament for treating of a disease in an eyeball comprising an RNA-encapsulated liposome, the RNA-encapsulated liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
wherein the bilayer lipid membrane is a bilayer lipid membrane comprising a neutral lipid, and a lipid derivative, a fatty acid derivative or an aliphatic hydrocarbon derivative of a water-soluble substance as constituent components.

21. The medicament for treating of a disease in an eyeball according to claim 19 or 20, wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyeth yl ammonium bromide and 3β-[N-(N',N'-dimethylaminoethyl) carbamoyl]cholesterol.

22. The medicament for treating of a disease in an eyeball according to any one of claims 19 to 21, wherein the lipid derivative, the fatty acid derivative or the aliphatic hydrocarbon derivative of a water-soluble substance is polyethylene glycol phosphatidyl ethanolamine.

23. The medicament for treating of a disease in an eyeball according to any one of claims 19 to 22, wherein the neutral lipid is egg yolk phosphatidylcholine.

24. The medicament for treating of a disease in an eyeball according to any one of claims 16 to 23, wherein the RNA is RNA having an action of suppressing the expression of the target gene utilizing RNA interference (RNAi).

25. The medicament for treating of a disease in an eyeball according to any one of claims 16 to 23, wherein the target gene associated with a disease in an eyeball is a gene involved in angiogenesis in an eye disease.

26. The medicament for treating of a disease in an eyeball according to any one of claims 16 to 23, wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.

27. The medicament for treating of a disease in an eyeball according to any one of claims 16 to 23, wherein the mRNA of a target gene associated with a disease in an eyeball is KLF5 mRNA.

28. The medicament for treating of a disease in an eyeball according to any one of claims 16 to 27, wherein the mRNA is either human or mouse mRNA.

29. A method of treating a disease in an eyeball, comprising administering a composition comprising an RNA-encapsulated liposome to a mammal, the liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
wherein the constituent components of the bilayer lipid membrane are soluble in a polar organic solvent, and
wherein the constituent components of the bilayer lipid membrane and the complex particle are dispersible in a liquid comprising the polar organic solvent at a specific concentration.

30. The method of treating a disease in an eyeball according to claim 29, wherein the polar organic solvent is an alcohol.

31. The method of treating a disease in an eyeball according to claim 29, wherein the polar organic solvent is ethanol.

32. The method of treating a disease in an eyeball according to any one of claims 29 to 31, wherein the lead particle is a lead particle comprising a cationic lipid, and
wherein the bilayer lipid membrane is a bilayer lipid membrane comprising a neutral lipid, and a lipid derivative, a fatty acid derivative or an aliphatic hydrocarbon derivative of a water-soluble substance as constituent components.

33. A method of treating a disease in an eyeball, comprising administering a composition comprising an RNA-encapsulated liposome to a mammal, the liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
wherein the bilayer lipid membrane is a bilayer lipid membrane comprising a neutral lipid, and a lipid derivative, a fatty acid derivative or an aliphatic hydrocarbon derivative of a water-soluble substance as constituent components.

34. The method of treating a disease in an eyeball according to claim 32 or 33, wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.

35. The method of treating a disease in an eyeball according to any one of claims 32 to 34, wherein the lipid derivative, the fatty acid derivative or the aliphatic hydrocarbon derivative of a water-soluble substance is polyethylene glycol phosphatidyl ethanolamine.

36. The method of treating a disease in an eyeball according to any one of claims 32 to 35, wherein the neutral lipid is egg yolk phosphatidylcholine.

37. The method of treating a disease in an eyeball according to any one of claims 29 to 36, wherein the RNA is RNA having an action of suppressing the expression of the target gene utilizing RNA interference (RNAi).

38. The method of treating a disease in an eyeball according to any one of claims 29 to 36, wherein the target gene associated with a disease in an eyeball is a gene involved in angiogenesis in an eye disease.

39. The method of treating a disease in an eyeball according to any one of claims 29 to 36, wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.

40. The method of treating a disease in an eyeball according to any one of claims 29 to 36, wherein the mRNA of a target gene associated with a disease in an eyeball is KLF5 mRNA.

41. The method of treating a disease in an eyeball according to any one of claims 29 to 40, wherein the mRNA is either human or mouse mRNA.

42. Use of a composition comprising an RNA-encapsulated liposome for production of a medicament for treating of a disease in an eyeball, the liposome composing a complex particle comprising a lead particle and the RNA as constituent components, the RNA comprising a sequence being 15 to 30 contiguous nucleotides of mRNA of a target gene associated with a disease in an eyeball and a sequence complementary to the sequence; and a bilayer lipid membrane for coating the complex particle;
wherein the constituent components of the bilayer lipid membrane are soluble in a polar organic solvent, and
wherein the constituent components of the bilayer lipid membrane and the complex particle are dispersible in a liquid comprising the polar organic solvent at a specific concentration.

43. The use according to claims 42, wherein the polar organic solvent is an alcohol.

44. The use according to claim 42, wherein the polar organic solvent is ethanol.

45. The use according to any one of claims 42 to 44 , wherein the lead particle is a lead particle comprising a cationic lipid, and
wherein the bilayer lipid membrane is a bilayer lipid membrane comprising a neutral lipid, and a lipid derivative, a fatty acid derivative or an aliphatic hydrocarbon derivative of a water-soluble substance as constituent components.

46. Use of a composition comprising an RNA-encapsulated liposome for production of a medicament for treating of a disease in an eyeball, the liposome composing a complex particle comprising a lead particle and the RNA as constituent components, the RNA comprising a sequence being 15 to 30 contiguous nucleotides of mRNA of a target gene and a sequence complementary to the sequence, the lead particle comprising a cationic lipid; and a bilayer lipid membrane for coating the complex particle;
wherein the bilayer lipid membrane is a bilayer lipid membrane comprising a neutral lipid, and a lipid derivative, a fatty acid derivative or an aliphatic hydrocarbon derivative of a water-soluble substance as constituent components.

47. The use according to claim 45 or 46, wherein the cationic lipid is one or more compounds selected from N-[1-(2,3-dioleoylpropyl)]-N,N,N-trimethyl ammonium chloride, N-[1-(2,3-dioleoylpropyl)]-N,N-dimethylamine, N-[1-(2,3-dioleyloxypropyl)]-N,N,N-trimethyl ammonium chloride, N-[1-(2,3-ditetradecyloxypropyl)]-N,N-dimethyl-N-hydroxyethyl ammonium bromide and 3β-[N-(N',N'-dimethylaminoethyl) carbamoyl]cholesterol.

48. The use according to any one of claims 45 to 47 , wherein the lipid derivative, the fatty acid derivative or the aliphatic hydrocarbon derivative of a water-soluble substance is polyethylene glycol phosphatidyl ethanolamine.

49. The use according to any one of claims 45 to 48, wherein the neutral lipid is egg yolk phosphatidylcholine.

50. The use according to any one of claims 42 to 49 , wherein the RNA is RNA having an action of suppressing the expression of the target gene utilizing RNA interference (RNAi).

51. The use according to any one of claims 42 to 49 , wherein the target gene associated with a disease in an eyeball is a gene involved in angiogenesis in an eye disease.

52. The use according to any one of claims 42 to 49 , wherein the target gene associated with a disease in an eyeball is a gene encoded for any one selected from vascular endothelial growth factor, vascular endothelial growth factor receptor, fibroblast growth factor, fibroblast growth factor receptor, platelet-derived growth factor, platelet-derived growth factor receptor, hepatocyte growth factor, hepatocyte growth factor receptor, Kruppel-like factor, Ets transcription factor, nuclear factor and hypoxia-inducible factor.

53. The use according to any one of claims 42 to 49 , wherein the mRNA of a target gene associated with a disease in an eyeball is KLF5 mRNA.

54. The use according to any one of claims 42 to 53 , wherein the mRNA is either human or mouse mRNA.
